(19) Europäisches Patentamt
European Patent Office
Office européen des brevets

(11) Veröffentlichungsnummer: **0 154 849**
**B1**

(12) # EUROPÄISCHE PATENTSCHRIFT

(45) Veröffentlichungstag der Patentschrift: **25.01.89**

(21) Anmeldenummer: **85101858.0**

(22) Anmeldetag: **21.02.85**

(51) Int. Cl.⁴: **C 09 K 19/12**, C 09 K 19/30, C 07 C 13/18, C 07 C 13/28, C 07 C 23/10, C 07 C 23/18, C 07 C 43/162, C 07 C 47/105, C 07 C 69/75, C 07 C 121/46

(54) **Cyclohexanderivate.**

(30) Priorität: **27.02.84 DE 3407013**

(43) Veröffentlichungstag der Anmeldung: **18.09.85 Patentblatt 85/38**

(45) Bekanntmachung des Hinweises auf die Patenterteilung: **25.01.89 Patentblatt 89/4**

(84) Benannte Vertragsstaaten: **CH DE FR GB LI**

(56) Entgegenhaltungen:
**EP-A-0 056 501**
**EP-A-0 063 003**
**EP-A-0 087 963**
**EP-A-0 107 759**

**Die Akte enthält technische Angaben, die nach dem Eingang der Anmeldung eingereicht wurden und die nicht in dieser Patentschrift enthalten sind.**

(73) Patentinhaber: **Merck Patent Gesellschaft mit beschränkter Haftung, Frankfurter Strasse 250, D-6100 Darmstadt (DE)**

(72) Erfinder: **Eidenschink, Rudolf, Dr., Kornblumenstrasse 1, D-6115 Münster (DE)**
Erfinder: **Krause, Joachim, Dr., Samuel- Morse-Strasse 14, D-6110 Dieburg (DE)**
Erfinder: **Wächtler, Andreas, Dr., Goethestrasse 34, D-6103 Griesheim (DE)**
Erfinder: **Hittich, Reinhard, Dr., Am Kirchberg 11, D-6101 Modautal 1 (DE)**
Erfinder: **Scheuble, Bernhard, Dr., Am Grenzweg 18, D-6148 Alsbach (DE)**
Erfinder: **Weber, Georg, Wilhelm- Leuschner-Strasse 38, D-6106 Erzhausen (DE)**
Erfinder: **Kurmeier, Hans- Adolf, Dr., Hinter der Schule 3a, D-6104 Seeheim- Jugenheim (DE)**

**EP 0 154 849 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Cyclohexanderivate der Formel II

$R^{1'}$-A-$Z^0$ -($A^{1'}$ -$Z^{1'}$)$_m$-($A^{2'}$)$_n$-$R^{2'}$ II

worin

| | |
|---|---|
| $R^{1'}$ und $R^{2'}$ | jeweils H, eine Alkylgruppe mit 1 - 10 C-Atomen worin auch eine oder zwei nicht benachbarte $CH_2$-Gruppen durch O-Atome oder -CH=CH- ersetzt sein können, F, Cl, Br, CN, -COOR, -O-COR, SOR, $SO_2R$ oder -C≡C-$R^{3'}$, |
| $A^{1'}$ und $A^{2'}$ | jeweils unsubstituierte oder durch ein oder zwei F- und/oder Cl-Atome und/oder $CH_3$-Gruppen und/oder CN-Gruppen substituierte 1,4-Phenylen-, 1,4-Cyclohexylen-, 1,3-Dioxan-2,5-diyl-, 1,3-Dithian-2,5-diyl-, Piperidin-1,4-diyl-, 1,4-Bicyclo(2,2,2)octylen-, Pyridazin-3,6-diyl- oder Pyrimidin-2,5-diylgruppen oder A, |
| A | eine in 1- und/oder 4-Stellung durch unsubstituiertes oder substituiertes Alkyl oder fluoriertes Alkyl mit jeweils 1 - 5 C-Atomen, worin auch eine oder zwei nicht benachbarte $CH_2$-Gruppen durch eine Gruppierung aus der Gruppe -O-, -CO-, -O-CO-, -CO-O-, -C≡C-, -CH=CH-, -S-, -SO- und -$SO_2$- ersetzt sein können, F, Cl, Br, CN und/oder -CHO substituierte 1,4-Cyclohexylengruppe, die 1 oder 2 weitere Substituenten tragen kann, |
| $Z^0$ und $Z^{1'}$ | jeweils -CO-O-, -O-CO-, -$CH_2CH_2$-, -$OCH_2$-, -$CH_2O$- oder eine Einfachbindung, |
| $R^{3'}$ | H, CN oder eine Alkylgruppe mit 1 - 7 C-Atomen, |
| R | eine Alkylgruppe mit 1 - 10 C-Atomen, und |
| m und n | jeweils unabhängig voneinander 1 oder 2 |

bedeuten, und
(m + n) ≧ 3, oder falls $Z^0$ eine Einfachbindung bedeutet und $Z^{1'}$ keine Einfachbindung bedeutet, auch ≧ 2 ist, wobei für
m = 2 und/oder n = 2 die Gruppen $A^{1'}$ und/oder $A^{2'}$ und/oder $Z^{1'}$ gleich oder voneinander verschieden sein können,

sowie Cyclohexanderivate der Formeln

| | |
|---|---|
| Alkyl-Phe-Phe-A-Alkyl | VIII 32 |
| Alkoxy-Phe-Phe-A-Alkyl | VIII 33 |
| Alkyl-Phe-Phe-CO-O-A-Alkyl | VIII 53 |
| Alkoxy-Phe-Phe-CO-O-A-Alkyl | VIII 54 |
| Alkyl-Phe-Phe-O-CO-A-Alkyl | VIII 55 |
| Alkoxy-Phe-Phe-O-CO-A-Alkyl | VIII 56 |

worin -A- 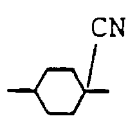

und Phe jeweils unsubstituiertes oder durch ein oder zwei F- und/oder Cl-Atome und/oder $CH_3$-Gruppen und/oder CN-Gruppen substituiertes 1,4-Phenylen bedeutet
und Alkyl und Alkoxy 1 bis 10 C-Atome haben.

Der Einfachheit halber bedeuten im folgenden
Phe eine insubstituierte oder substituierte 1,4-Phenylengruppe,
Cy eine 1,4-Cyclohexylengruppe,
Dio eine 1,3-Dioxan-2,5-diylgruppe,
Dit eine 1,3-Dithian-2,5-diylgruppe,
Bi eine Bicyclo(2,2,2)-octylengruppe,
Pip eine Piperidin-1,4-diylgruppe,
Pyn eine Pyridazin-3,6-diylgruppe und
Pyr eine Pyrimidin-2,5-diylgruppe.

Die erfindungsgemäßen können als Komponenten flüssigkristalliner Phasen verwendet werden, insbesondere fur Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Phasen geeignet sind.

Es wurde gefunden, daß die erfindungsgemäßen Verbindungen als Komponenten flüssigkristalliner Phasen vorzüglich geeignet sind. Insbesondere sind mit ihrer Hilfe stabile flüssigkristalline Phasen mit stark negativer dielektrischer Anisotropie und damit kleiner Schwellen- bzw. Steuerspannung elektrooptischer Effekte, sehr kleiner optischer Anisotropie und vergleichsweise niedriger Viskosität herstellbar.

Überraschenderweise zeigte sich beim Zusatz von erfindungsgemäßen Verbindungen zu Mischungen mit positiver dielektrischer Anisotropie, daß selbst größere Zusätze (z. B. von 30 %) die Schwellenspannung nur unwesentlich erhöhen. Völlig unerwartet tritt gleichzeitig eine erhebliche Verbesserung der Kennliniensteilheit der Mischung auf, so daß der erfindungsgemäßen Verbindungen als besonders vorteilhaft geeignete Substanzen zur Herstellung von flüssigkristallinen Mischungen mit steiler Kennlinien anzusehen sind. Sie ermöglichen damit die Entwicklung hoch multiplexbarer Mischungen sehr kleiner optischer Anisotropie mit denen eine Drehzelle insbesondere im ersten Transmissionsminimum nach Gooch-Tarry betrieben werden kann. Damit ergibt sich eine sehr kleine Beobachtungswinkel-Abhängigkeit des Kontrastes.

Mit der Bereitstellung der erfindungsgemäßen Verbindungen wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die erfindungsgemäßen Verbindungen besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch erfindungsgemäße Verbindungen flüssigkristallinen Basismaterialien aus anderen Verbindungsklassen zugesetzt werden, um beispielsweise die dielektrische und/oder optische Anisotropie eines solchen Dielektrikums zu senken. Die erfindungsgemäßen Verbindungen eignen sich ferner als Zwischenprodukte zur Herstellung anderer Substanzen, die sich als Bestandteile flüssigkristalliner Phasen verwenden lassen.

Die erfindungsgemäßen sind in reinem Zustand farblos und bilden flüssigkristalline Mesophasen in einen für die elektrooptische Verwendung günstig gelegenen Temperaturbereich. Chemisch, thermisch und gegen Licht sind sie sehr stabil.

Gegenstand der Erfindung sind somit die Verbindungen der Formel II sowie die Cyclohexanderivate der Formeln VIII 32, VIII 33 und VIII 53 bis VIII 56, sowie ein Verfahren zu ihrer Herstellung, dadurch gekennzeichnet daß man eine Verbindungen, die sonst der erfindungsgemäßen Verbindung entspricht, aber an Stelle von H-Atomen eine oder mehrere reduzierbare Gruppen und/oder C-C-Bindungen enthält, mit einem reduzierenden Mittel behandelt,

oder daß man an eine Verbindung, die sonst der Formel II entspricht aber an Stelle des Restes A eine 1-Cyclohexen-1 4-diylgruppe enthält, die 1 oder 2 weitere F-, Cl- oder Br-Atome und/oder CN-Gruppen tragen kann, eine Verbindung der Formel HX (worin X F, Cl, Br oder CN bedeutet) anlagert,

oder daß man zur Herstellung von Estern der Formel II (worin $R^{1'}$ und/oder $R^{2'}$ -OCOR und/oder -COOR bedeuten und/oder worin $Z^0$ und/oder $Z^{1'}$ -CO-O- oder -O-CO- bedeuten), VIII 53, VIII 54, VIII 55 oder VIII 56 eine entsprechende Carbonsäure oder eines ihrer reaktionsfähigen Derivate mit einem entsprechenden Alkohol oder einem seiner reaktionsfähigen Derivate umsetzt,

oder daß man zur Herstellung von Dioxanderivaten bzw. Dithianderivaten der Formel II (worin $A^{1'}$ und/oder $A^{2'}$ 1,3-Dioxan-2,5-diyl bzw. 1,3-Dithian-2,5-diyl bedeuten) einen entsprechenden Aldehyd oder eines seiner reaktionsfähigen Derivate mit einem entsprechenden Diol bzw. Dithiol umsetzt,

oder daß man zur Herstellung von Nitrilen der Formel II (worin $R^{1'}$ und/oder $R^{2'}$ CN bedeuten und/oder worin A und/oder $A^{1'}$ und/oder $A^{2'}$ durch mindestens eine CN-Gruppe substituiert sind) ein entsprechendes Carbonsäureamid dehydratisiert oder ein entsprechendes Carbonsäurehalogenid mit Sulfamid umsetzt,

oder daß man zur Herstellung von Nitrilen der Formel II (worin A eine in 1- oder 4-Stellung durch CN substituierte 1,4-Cyclohexylengruppe bedeutet) ein Acetonitril der Formel III

$$E^1\text{-}CH_2CN \qquad \text{III}$$

worin

$E^1$ a) $R^{1'}$- oder
b) $R^{2'}\text{-}(A^{2'})_n\text{-}(Z^{1'}\text{-}A^{1'})_m\text{-}Z^0\text{-}$

bedeutet und
$R^{1'}$, $R^{2'}$, $A^{1'}$, $A^{2'}$, $Z^{1'}$, $Z^0$, m und n die angegebenen Bedeutungen haben,
mit einer Verbindung der Formel IV

$$(X^1\text{-}CH_2\text{-}CH_2)_2CH\text{-}E^2 \qquad \text{IV}$$

worin

$X^1$ Cl, Br, J, OH oder eine reaktionsfähig veresterte OH-Gruppe bedeutet,
$E^2$ a) $R^{2'}\text{-}(A^{2'})_n\text{-}(Z^{1'}\text{-}A^{1'})_m\text{-}Z^0\text{-}$ oder
b) $R^{1'}$-

bedeutet und
$R^{1'}$, $R^{2'}$, $A^{1'}$, $A^{2'}$, $Z^{1'}$, $Z^0$, m und n die angegebenen Bedeutungen haben,
umsetzt, oder daß man zur Herstellung von Nitrilen der Formel II (worin A eine in 1- oder 4-Stellung durch CN substituierte 1,4-Cyclohexylengruppe bedeutet, die zusätzlich durch ein oder zwei F-Atome und/oder CN-Gruppen substituiert sein kann; worin ferner bei (a) $Z^0$ eine Einfachbindung bedeutet) ein Nitril der Formel V

$Q^1$-$Q^3$-CN V

worin

| | |
|---|---|
| $Q^1$ | (a) $R^{1'}$- oder<br>(b) $R^{2'}$-$(A^{2'})_n$-$(Z^{1'}$-$A^{1'})_m$-$Z^0$ - und |
| $Q^3$ | eine unsubstituierte oder eine ein- oder zweifach durch F und/oder CN substituierte 1,4-Cyclohexylengruppe |

bedeuten und
$R^{1'}$, $R^{2'}$, $A^{1'}$, $A^{2'}$, $Z^{1'}$, $Z^0$, m und n die angegebenen Bedeutungen haben
mit einer Verbindung der Formel VI

$Q^2$-$X^1$ VI

worin

| | |
|---|---|
| $Q^2$ | a) $R^{2'}$-$(A^{2'})_n$-$(Z^{1'}$-$A^{1'})_m$- oder<br>b) $R^{1'}$- |

bedeuten und
$X^{1'}$, $R^{1'}$, $R^{2'}$, $A^{1'}$, $A^{2'}$, $Z^{1'}$, m und n die angegebeneb Bedeutungen haben,
umsetzt,

oder daß man zur Herstellung von Ethern der Formel II (worin $R^{1'}$ und/oder $R^{2'}$ eine Alkylgruppe bedeuten, worin eine oder zwei $CH_2$-Gruppen durch O-Atome ersetzt sind und/oder $Z^0$ und/oder $Z^{1'}$ eine $-OCH_2-$ oder $-CH_2O-$Gruppe sind) eine entsprechende Hydroxyverbindung verethert,

oder daß man zur Herstellung von Verbindungen der Formel II, worin $R^{1'}$ und/oder $R^{2'}$ SOR bzw $SO_2R$ bedeuten, eine entsprechende Verbindung worin $R^1$ und/oder $R^2$ SR bzw. SOR bedeuten, oxidiert,

oder daß man zur Herstellung von Verbindungen der Formel II, die $CF_3$-Gruppen enthalten, eine entsprechende Carbonsäure mit $SF_4$ umsetzt,

und/oder daß man gegegenenfalls eine Chlor- oder Bromverbindung der Formel II (worin $R^{1'}$ und/oder $R^{2'}$ Cl oder Br bedeuten und/oder worin A durch mindestens ein Chlor- oder Bromatom substituiert ist und/oder $A^{1'}$ und/oder $A^{2'}$ durch mindestens ein Chloratom substituiert sind) mit einem Cyanid umsetzt,

und/oder daß man gegebenenfalls eine Base der Formel II durch Behandeln mit einer Säure in eines ihrer Säureadditionssalze umwandelt,

oder daß man gegebenenfalls eine Verbindung der Formel II aus einem ihrer Säureadditionssalze durch Behandeln mit einer Base freisetzt,

oder daß man zur Herstellung von Nitrilen der Formeln VIII 32 oder VIII 33 ein entsprechendes 4-substituiertes Cyclohexancarbonitril mitr einer entsprechenden Halogenverbindung, Hydroxyverbindung oder einem ihrer reaktionsfähigen Derivate umsetzt.

Weiterhin ist Gegenstand der Erfindung die Verwendung der Verbindungen fer Formel II sowie VIII 32, VIII 33, VIII 53, VIII 54, VIII 55 und VIII 56 als Komponenten flüssigkristalliner Phasen.

Gegenstand der Erfindung sind ferner flüssigkristalline Phasen mit mindestens zwei flüssigkristallinen Komponenten, dadurch gekennzeichnet, daß mindestens eine Komponente eine Verbindung der Formel II, VIII 32, VIII 33, VIII 53, VIII 54, VIII 55 oder VIII 56 ist, sowie Flüssigkristall-Anzeigeelemente, insbesondere elektrooptische Anzeigeelemente, die derartige Phasen enthalten.

Vor- und nachstehend haben $R^{1'}$, $R^{2'}$, $R^{3'}$, R, $A^{1'}$, $A^{2'}$, A, $Z^0$, $Z^{1'}$, m, n, $X^1$, $E^1$, $E^2$, $Q^1$, $Q^2$ und $Q^3$ die angegebene Bedeutung sofern nicht ausdrücklich etwas anderes vermerkt ist.

Die Verbindungen der Formel II umfassen insbesondere Verbindungen der Teilformel IIa (mit drei Ringen), IIb - IIg (mit vier Ringen), sowie IIh und IIi (mit fünf Ringen):

$R^{1'}$-A-$A^{1'}$-$Z^{1'}$-$A^{2'}$-$R^{2'}$ IIa

$R^{1'}$-A-$(A^{1'})_2$-$A^{2'}$-$R^{2'}$ IIb
$R^{1'}$-A-$A^{1'}$-$Z^{1'}$-$(A^{2'})_2$-$R^{2'}$ IIc
$R^{1'}$-A-$(A^{1'})_2$-$Z^{1'}$-$A^{2'}$-$R^{2'}$ IId
$R^{1'}$-A-$Z^0$-$(A^{1'})_2$-$A^{2'}$-$R^{2'}$ IIe
$R^{1'}$-A-$Z^0$-$(A^{1'})_2$-$Z^{1'}$-$A^{2'}$-$R^{2'}$ IIf
$R^{1'}$-A-$(A^{1'}$-$Z^{1'})_2$-$A^{2'}$-$R^{2'}$ IIg

$R^{1'}$-A-$(A^{1'})_2$-$(A^{2'})_2$-$R^{2'}$ IIh
$R^{1'}$-A-$(A^{1'})_2$-$Z^{1'}$-$(A^{2'})_2$-$R^{2'}$ IIi

Darunter sind diejenigen der Teilformeln IIa, IIb, IIc, IId und IIe bevorzugt. Die bevorzugten Verbindungen der Teilformel IIa umfassen solche der Teilformel IIa1 bis IIa25:

$R^{1'}$-A-Phe-$Z^{1'}$-Phe-$R^{2'}$ IIa1
$R^{1'}$-A-Phe-$Z^{1'}$-Cy-$R^{2'}$ IIa2
$R^{1'}$-A-Cy-$Z^{1'}$-Cy-$R^{2'}$ IIa3
$R^{1'}$-A-Cy-$Z^{1'}$-Phe-$R^{2'}$ IIa4
$R^{1'}$-A-Pyr-$Z^{1'}$-Phe-$R^{2'}$ IIa5
$R^{1'}$-A-Pyr-$Z^{1'}$-Cy-$R^{2'}$ IIa6
$R^{1'}$-A-Cy-$Z^{1'}$-Dio-$R^{2'}$ IIa7
$R^{1'}$-A-Cy-$Z^{1'}$-Dit-$R^{2'}$ IIa8
$R^{1'}$-A-Cy-$Z^{1'}$-Pyr-$R^{2'}$ IIa9
$R^{1'}$-A-Cy-$Z^{1'}$-Pyn-$R^{2'}$ IIa10
$R^{1'}$-A-Cy-$Z^{1'}$-Bi-$R^{2'}$ IIa11
$R^{1'}$-A-Phe-$Z^{1'}$-Dio-$R^{2'}$ IIa12
$R^{1'}$-A-Phe-$Z^{1'}$-Dit-$R^{2'}$ IIa13
$R^{1'}$-A-Phe-$Z^{1'}$-Bi-$R^{2'}$ IIa14
$R^{1'}$-A-Phe-$Z^{1'}$-Pyn-$R^{2'}$ IIa15
$R^{1'}$-A-Dio-$Z^{1'}$-Phe-$R^{2'}$ IIa16
$R^{1'}$-A-Dio-$Z^{1'}$-Cy-$R^{2'}$ IIa17
$R^{1'}$-A-Dit-$Z^{1'}$-Phe-$R^{2'}$ IIa18
$R^{1'}$-A-Dit-$Z^{1'}$-Cy-$R^{2'}$ IIa19
$R^{1'}$-A-Bi-$Z^{1'}$-Phe-$R^{2'}$ IIa20
$R^{1'}$-A-Bi-$Z^{1'}$-Cy-$R^{2'}$ IIa21
$R^{1'}$-A-Pyr-$Z^{1'}$-Cy-$R^{2'}$ IIa22
$R^{1'}$-A-Pyr-$Z^{1'}$-Phe-$R^{2'}$ IIa23
$R^{1'}$-A-Pyn-$Z^{1'}$-Phe-$R^{2'}$ IIa24
$R^{1'}$-A-Pyn-$Z^{1'}$-Cy-$R^{2'}$ IIa25

Darunter sind diejnigen der Formeln IIa1 bis IIa4, insbesondere IIa1 und IIa2, besonders bevorzugt.
In den Verbindungen der Teilformel IIa ist $Z^{1'}$ bzw. $Z^0$ vorzugsweise -CO-O-, -O-CO- oder -$CH_2CH_2$-.

Die bevorzugten Verbindungen der Teilformel IIb umfassen solche der Teilformeln IIb1 bis IIb16:

$R^{1'}$-A-Phe-Phe-Cy-$R^{2'}$ IIb1
$R^{1'}$-A-Phe-Phe-Dio-$R^{2'}$ IIb2
$R^{1'}$-A-Phe-Phe-A-$R^{2'}$ IIb3
$R^{1'}$-A-Cy-Phe-Phe-$R^{2'}$ IIb4
$R^{1'}$-A-Cy-Phe-Cy-$R^{2'}$ IIb5
$R^{1'}$-A-Cy-Phe-Dio-$R^{2'}$ IIb6
$R^{1'}$-A-Phe-Phe-Dit-$R^{2'}$ IIb7
$R^{1'}$-A-Phe-Phe-Bi-$R^{2'}$ IIb8
$R^{1'}$-A-Phe-Cy-Cy-$R^{2'}$ IIb9
$R^{1'}$-A-Phe-Cy-Dio-$R^{2'}$ IIb10
$R^{1'}$-A-Phe-Cy-Dit-$R^{2'}$ IIb11
$R^{1'}$-A-Phe-Pyr-Cy-$R^{2'}$ IIb12
$R^{1'}$-A-Phe-Pyn-Cy-$R^{2'}$ IIb13
$R^{1'}$-A-Pyr-Phe-Cy-$R^{2'}$ IIb14
$R^{1'}$-A-Pyn-Phe-Cy-$R^{2'}$ IIb15
$R^{1'}$-A-Pyn-Phe-Dio-$R^{2'}$ IIb16

Darunter sind diejenigen der Formeln IIb1 bis IIb4 besonders bevorzugt.
Insbesondere bevorzugt sind weiterhin Verbindungen der Formel IIb, worin mindestens einer der Ringe $A^{1'}$ vorzugsweise durch F substituiert ist.

Die bevorzugten Verbindungen der Teilformel IIc umfassen solche, worin $A^{1'}$ und $A^{2'}$ aus der Reihe Cy, Phe, Dio ausgewählt sind und $Z^{1'}$ -CO-O-, -O-CO- oder -$CH_2CH_2$- bedeutet.

Die bevorzugten Verbindungen der Teilformel IId umfassen solche der Teilformeln IId1 bis IId8:

$R^{1'}$-A-Phe-Phe-$Z^{1'}$-Cy-$R^{2'}$ IId1
$R^{1'}$-A-Phe-Phe-$Z^{1'}$-Dio-$R^{2'}$ IId2

$R^{1'}$-A-Phe-Phe-$Z^{1'}$-Phe-$R^{2'}$ IId3
$R^{1'}$-A-Cy-Phe-$Z^{1'}$-Cy-$R^{2'}$ IId4
$R^{1'}$-A-Cy-Phe-$Z^{1'}$-Phe-$R^{2'}$ IId5
$R^{1'}$-A-Cy-Cy-$Z^{1'}$-Phe-$R^{2'}$ IId6
$R^{1'}$-A-Cy-Cy-$Z^{1'}$-Cy-$R^{2'}$ IId7
$R^{1'}$-A-Cy-Phe-$Z^{1'}$-Dio-$R^{2'}$ IId8

Die bevorzugten Verbindungen der Teilformel IIe umfassen solche der Teilformeln IIe1 bis IIe8:

$R^{1'}$-A-$Z^0$-Phe-Phe-Cy-$R^{2'}$ IIe1
$R^{1'}$-A-$Z^0$-Cy-Phe-Phe-$R^{2'}$ IIe2
$R^{1'}$-A-$Z^0$-Cy-Phe-Cy-$R^{2'}$ IIe3
$R^{1'}$-A-$Z^0$-Phe-Phe-Dio-$R^{2'}$ IIe4
$R^{1'}$-A-$Z^0$-Cy-Cy-Phe-$R^{2'}$ IIe5
$R^{1'}$-A-$Z^0$-Phe-Cy-Cy-$R^{2'}$ IIe6
$R^{1'}$-A-$Z^0$-Dio-Cy-Phe-$R^{2'}$ IIe7
$R^{1'}$-A-$Z^0$-Phe-Cy-Dio-$R^{2'}$ IIe8

Darunter sind diejenigen der Formeln IIe1, IIe4 und IIe6 besonders bevorzugt. Insbesondere bevorzugt sind weiterhin Verbindungen der Forneln IIe1 bis IIe8, worin mindestens einer der Ringe $A^{1'}$ substituiert ist, vorzugsweise durch F, und/oder solche, worin Z° -CO-O-, -O-CO- oder $-CH_2CH_2-$, besonders bevorzugt $-CH_2CH_2-$, bedeutet.

In der Verbindungen der vor- und nachstehenden Formeln beteuten $R^{1'}$ und $R^{2'}$ vorzugsweise Alkyl, ferner Alkoxy oder eine andere Oxaalkylgruppe. Weiterhin bevorzugt sind Verbindungen der Formel II worin einer der Reste $R^{1'}$ und $R^{2'}$, CN, F, Cl, Br, SOR oder $SO_2R$, insbesondere CN, F oder Cl, bedeutet.

$A^{1'}$ und $A^{2'}$ sind bevorzugt unabhängig voneinander Cy, Dio oder Phe, ferner bevorzugt Dit, Pyr oder Pip; bevorzugt enthält die Verbindung der Formel II nicht mehr als einen der Reste Dio, Dit, Pip, Bi, Pyn oder Pyr.

A ist bevorzugt eine 1-X-1,4-Cyclohexylengruppe, die keine weiteren Substituenten trägt und worin X Alkyl, Alkoxy, fluoriertes Alkyl oder fluoriertes Alkoxy mit jeweils 1 - 5 C-Atomen, F, Cl, Br oder CN bedeutet. Bevorzugt ist X eine CN-, $CH_3$-, $CH_3O$- oder $CF_3$-Gruppe. Besonders bevorzugt ist CN.

A ist ferner bevorzugt eine 1-X-1,4-Cyclohexylengruppe, die keine weiteren Substituenten trägt und worin X -CHO, -COO-Alkyl, -S-Alkyl, -SO-Alkyl, $-SO_2$-Alkyl bedeutet, worin Alkyl eine geradkettige Alkylgruppe mit 1 - 4 C-Atomen ist.

$Z^0$ und $Z^{1'}$ sind bevorzugt unabhängig voneinander Einfachbindungen oder $-CH_2CH_2-$, -CO-O- oder -O-CO-Gruppen.

Weitere Substituenten in A sind vorzugsweise $CH_3$, F oder CN.

$X^1$ ist vorzugsweise Cl oder Br, aber auch J, OH oder reaktionsfähig verestertes OH wie Alkylsulfonyloxy mit insbesondere 1 - 6 C-Atomen (z. B. Methylsulfonyloxy) oder Arylsulfonyloxy mit insbesondere 6 - 10 C-Atomen (z. B. Phenyl-, p-Tolyl- oder Naphthylsulfonyloxy).

Falls $R^{1'}$ und/oder $R^{2'}$ Alkylreste bedeuten, in denen auch eine ("Alkoxy" bzw. "Oxaalkyl") oder zwei ("Alkoxyalkoxy" bzw. "Dioxaalkyl") nicht benachbarte $CH_2$-Gruppen durch O-Atome ersetzt sein können, so können sie geradkettig oder verzweigt sein. Vorzugsweise sind sie geradkettig, haben 2, 3, 4, 5, 6 oder 7 C-Atome und bedeuten demnach bevorzugt Ethyl, Propyl, Butyl, Pentyl, Hexyl, Heptyl, Ethoxy, Propoxy, Butoxy, Pentoxy, Hexoxy, Heptoxy, 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, ferner Methyl, Octyl, Nonyl, Decyl, Methoxy, Octoxy, Nonoxy, Decoxy, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Verbindungen der Formel II mit verzweigten Flügelgruppen $R^{1'}$ bzw. $R^{2'}$ können gelegentlich wegen einer besseren Löslichkeit in den üblichen flüssigkristallinen Basismaterialien von Bedeutungen sein, insbesondere aber als chirale Dotierstoffe, wenn sie optisch aktiv sind.

Verzweigte Gruppen dieser Art enthalten in der Regel nicht mehr als eine Kettenverzweigung. Bevorzugte verzweigte Reste sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl.

Bei Verbindungen mit verzweigten Flügelgruppen umfasst Formel II sowohl die optischen Antipoden als auch Racemate sowie deren Gemische.

In den Resten R und X sind die Alkylgruppen bzw. Alkoxygruppen ebenfalls vorzugsweise geradkettig und bedeuten insbesondere Methyl oder Ethyl, ferner Propyl, Butyl oder Pentyl, X auch Methoxy oder Ethoxy, ferner Propoxy, Butoxy oder Pentoxy.

Unter den Verbindungen der Formel II und deren Unterformeln sind diejenigen bevorzugt, in denen mindestens einer der darin enthaltenen Reste eine der angegebenen bevorzugten Bedeutungen hat. Besonders

bevorzugte kleinere Gruppen von erfindungsgemäßen Verbindungen sind diejenigen der Formeln VIII 32, VIII 33, VIII 53, VIII 54, VIII 55, VIII 56 und VIII 57 - 81 (entsprechend Formel II):

| | |
|---|---|
| Alkyl-Phe-Phe-A-Alkyl | VIII 32 |
| Alkoxy-Phe-Phe-A-Alkyl | VIII 33 |
| Alkyl-Phe-Phe-CO-O-A-Alkyl | VIII 53 |
| Alkoxy-Phe-Phe-CO-O-A-Alkyl | VIII 54 |
| Alkyl-Phe-Phe-O-CO-A-Alkyl | VIII 55 |
| Alkoxy-Phe-Phe-O-CO-A-Alkyl | VIII 56 |
| Alkyl-A-Phe-Phe-Cy-Alkyl | VIII 57 |
| Alkyl-A-Phe-Phe-Dio-Alkyl | VIII 58 |
| Alkyl-A-Phe-Phe-Dio-Alkoxy | VIII 59 |
| Alkyl-A-Phe-Phe-A-Alkyl | VIII 60 |
| Alkyl-A-Cy-Phe-Phe-Alkyl | VIII 61 |
| Alkyl-A-Cy-Phe-Phe-Alkoxy | VIII 62 |
| Alkyl-A-Cy-Phe-Phe-CN | VIII 63 |
| Alkyl-A-Phe-$CH_2CH_2$-Phe-Alkyl | VIII 64 |
| Alkyl-A-Phe-$CH_2CH_2$-Phe-Alkoxy | VIII 65 |
| Alkyl-A-Phe-$CH_2CH_2$-Phe-CN | VIII 66 |
| Alkyl-A-Phe-$CH_2CH_2$-Cy-Alkyl | VIII 67 |
| Alkyl-A-Cy-$CH_2CH_2$-Cy-Alkyl | VIII 68 |
| Alkyl-A-Cy-$CH_2CH_2$-Phe-Alkyl | VIII 69 |
| Alkyl-A-Cy-$CH_2CH_2$-Phe-Alkoxy | VIII 70 |
| Alkyl-A-Cy-$CH_2CH_2$-Phe-CN | VIII 71 |
| Alkyl-A-Phe-COO-Phe-Alkyl | VIII 72 |
| Alkyl-A-Phe-COO-Phe-Alkoxy | VIII 73 |
| Alkyl-A-Phe-COO-Phe-CN | VIII 74 |
| Alkyl-A-Phe-OCO-Phe-Alkyl | VIII 75 |
| Alkyl-A-Phe-OCO-Phe-Alkoxy | VIII 76 |
| Alkyl-A-Phe-OCO-Phe-CN | VIII 77 |
| Alkyl-A-Phe-COO-Cy-Alkyl | VIII 78 |
| Alkyl-A-Phe-OCO-Cy-Alkyl | VIII 79 |
| Alkyl-A-$CH_2CH_2$-Phe-Phe-Cy-Alkyl | VIII 80 |
| Alkyl-A-Phe-Phe-$CH_2CH_2$-Cy-Alkyl | VIII 81 |

In den Verbindungen VIII 32, 33 und 53 bis 81 bedeutet Alkyl bzw. Alkoxy vorzugsweise eine geradkettige Alkyl- bzw. Alkoxygruppe mit jeweils 2 bis 7 C-Atomen. VIII 32 und VIII 33, sind besonders bevorzugt.

Verbindungen der Formel II (worin einer der Reste $R^{1'}$, $R^{2'}$ oder $R^{3'}$ F, Cl, Br oder CN, insbesondere CN, bedeutet) worin A jeweils eine in 1- und/oder 4-Stellung durch unsubstituiertes oder substituiertes Alkyl oder fluoriertes Alkyl mit jeweils 1 - 5 C-Atomen, worin auch eine oder zwei nicht benachbarte $CH_2$-Gruppen durch eine Gruppierung aus der Gruppe -O-, -CO-, -O-CO-, -CO-O-, -CH=CH-, -S-, -SO- und -$SO_2$- ersetzt sein können, und/oder -CHO substituierte 1,4-Cyclohexylengruppe bedeutet, eignen sich als Verbindungen mit positiver dielektrischer Anisotropie insbesondere als Komponenten flüssigkristalliner Phasen mit insgesamt positiver dielektrischer Anisatropie. A ist in diesem Zusammenhang vorzugsweise eine in 1- oder 4-Stellung durch Methyl substituierte 1,4-Cyclohexylengruppe (Formel (5) oder (6)).

Die erfindungsgemäßen flüssigkristallinen Phasen enthaltend diese positiven Verbindungen der Formel II enthalten vorzugsweise mindestens eine weitere Komponente ausgewählt aus den Verbindungen der folgenden Formeln,

Alkyl-Phe-Phe-CN
Alkyl-Cy-Phe-CN
Alkyl-Cy-Phe-Phe-CN
Alkyl-Cy-Cy-Phe-CN
Alkyl-Cy-Cy-Cy-CN
Alkyl-Cy-Cy-CN
Alkyl-Dio-Phe-CN

worin Alkyl jeweils n-Alkyl mit 2 bis 7 C-Atomen bedeutet.

Alkenylgruppen in den Verbindungen der Formel II sind vorzugsweise geradkettige trans-Alkenylgruppen der Formel

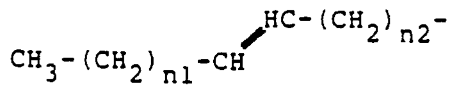

worin
n2 0 oder 2, vorzugsweise 2, und
n1 1 bis 5 bedeutet.

In den Verbindungen der vorstehenden Formeln VIII 32, 33 und 53 bis 81 insbesondere VIII 32, 33, 57, 58, 59, 60, 61, 62, und 63, kann Phe auch eine in 2- oder 3-Position lateral, insbesondere durch F, substituierte 1,4-Phenylengruppe bedeuten. In den Verbindungen der vorstehend genannten Formeln enthält die Gruppe A einen Substituenten X, der in 1- oder 4-Stellung stehen kann. So umschließen z. B. die Verbindungen der Formel II solche der nachstehenden Teilformeln II' und II''

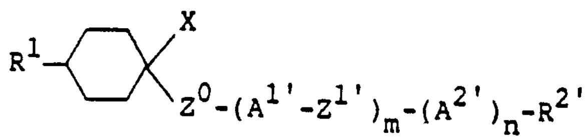 II'

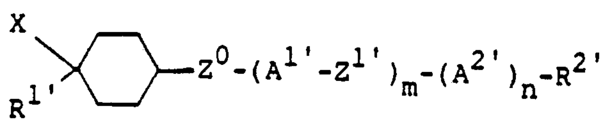 II''

(wobei der Cyclohexanring zusätzlich einen weiteren Substituenten X in der gegenüberliegenden Stellung (4- bzw. 1-Stellung) des Cyclohexanrings sowie 1 oder 2 weitere F-, Cl- oder Br-Atome und/oder CN-Gruppen tragen kann). In diesen Sinne sind weiterhin Verbindungen der angegebenen Formel II besonders bevorzugt, in denen der Rest A jeweils

(1) 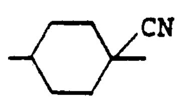 oder

(2) 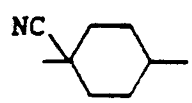 oder

(3) 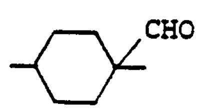 oder

(4) 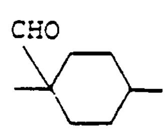 oder

(5) 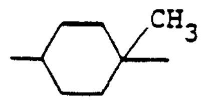 oder

(6) 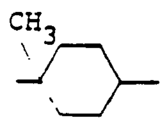 . oder

(7) 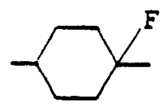 oder

(8) 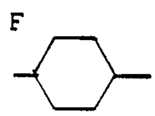 oder

(9) 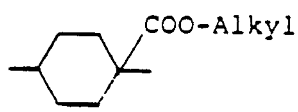 oder

(10) 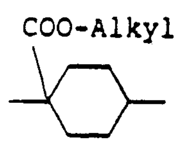 oder

(11) 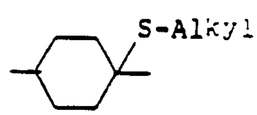 oder

(12) 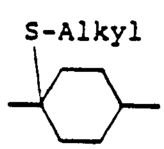 oder

(13) 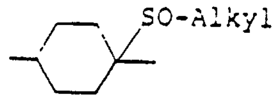 oder

(14) 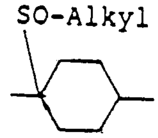 oder

(15) 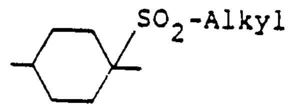 oder

(16) 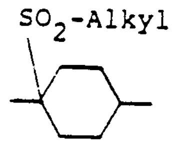 bedeutet,

wobei Alkyl jeweils eine geradkettige Alkylgruppe mit 1 - 4 C-Atomen ist.

Dabei sind diejenigen Stereoisomeren bevorzugt, in denen die Gruppen

$R^{1'}$ - und
$-Z^{0}-(A^{1'}-Z^{1'})_{m}-(A^{2'})_{n}-R^{2'}$

in trans-Stellung zueinander stehen, während der Substituent X in cis-Stellung zu der gegenüberstehenden Gruppe steht. So sind z. B. die nachstehenden Stereoisomeren der Verbindungen der Formel II' bevorzugt:

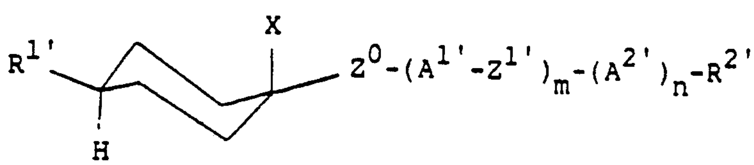

Allgemein sind diejenigen Verbindungen der Formel II bevorzugt, in denen im Ring A die Substituenten in 1- und 4-Stellung mit längster Kettenlänge trans-ständig sind. Falls bei der Synthese Gemische von Stereoisomeren anfallen, lassen sich die bevorzugten Isomeren durch an sich bekannte Trennverfahren, beispielsweise Chromatographie oder Kristallisation (gegebenenfalls in Gegenwart von Harnstoff) isolieren.

Diejenigen der vorstehend genannten Formeln, die eine oder mehrere der Gruppen Dio, Dit, Pip und/oder Pyr enthalten, umschließen jeweils die beiden möglichen 2,5- (Dio, Dit, Pyr) bzw. 1,4-Stellungsisomeren (Pip).

Die erfindungsmäßen Verbindungen werden nach an sich bekannten Methoden hergestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den erfindungsgemäßen Verbindungen umsetzt. So können die erfindungsgemäßen Verbindungen hergestellt werden, indem man eine Verbindung, die sonst erfindungsgemäßen Verbindung entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z. B. freie oder veresterte Hydroxygruppen oder aromatisch gebundene Halogenatome. Bevorzugte Ausgangsstoffe für die Reduktion entsprechen der erfindungsgemäßen Verbindung, können aber an Stelle eines Cyclohexanrings einen Cyclohexenring oder Cyclohexanonring und/oder an Stelle einer $-CH_2CH_2-$Gruppe eine $-CH=CH-$Gruppe und/oder an Stelle einer $-CH_2-$Gruppe eine -CO-Gruppe und/oder an Stelle eines H-Atoms eine freie oder eine funktionell (z. B. im Form ihres p-Toluolsulfonats) abgewandelte OH-Gruppe enthalten.

Die Reduktion kann z. B. erfolgen durch katalytische Hydrierung bei Temperaturen zwischen etwa 0° und etwa 200°C sowie Drucken zwischen etwa 1 und 200 bar in einem inerten Lösungsmittel, z. B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z. B. $PtO_2$, PdO), auf einem Träger (z. B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°C) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°C) zu den entsprechenden Verbindungen der Formel I, die Alkylgruppen und/oder $-CH_2CH_2-$Brücken enthalten, reduziert werden.

Weiterhin sind Reduktionen mit komplexen Hydriden möglich. Beispielsweise können Arylsulfonyloxygruppen mit $LiAlH_4$ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°C. Doppelbindungen können (auch in Gegenwart von CN-Gruppen!) mit $NaBH_4$ oder Tributylzinnhydrid in Methanol hydriert werden; so entstehen z. B. aus 1-Cyancyclohexenderivaten die entsprechenden Cyclohexanderivate.

In analoger Weise sind auch erfindungsgemäße Aldehyde (worin A eine in 1- oder 4-Stellung durch -CHO substituierte 1,4-Cyclohexylengruppe bedeutet, die zusätzlich ein oder zwei weitere Substituenten tragen kann) durch Reduktion entsprechender Nitrile erhältlich.

Erfindungsgemäße Verbindungen sind weiterhin erhältlich, indem man an ein entsprechendes Cyclohexenderivat (das der erfindungsgemäßen Verbindung entspricht, aber an Stelle des Restes A eine 1-Cyclohexen-1,4-diylgruppe enthält, die 1 oder 2 weitere F-, Cl- oder Br-Atome und/oder CN-Gruppen tragen kann) eine Verbindung der Formel HX (Fluor-, Chlor-, Brom- oder Cyanwasserstoff) anlagert.

Diese Anlagerung gelingt z. B. in Gegenwart eines inerten Lösungsmittels, z. B. eines halogenierten Kohlenwasserstoffs wie $CH_2Cl_2$ oder $CHCl_3$, eines Nitrils wie Acetonitril oder eines Amids wie Dimethylformamid (DMF) bei Temperaturen zwischen etwa -10 und +150°C und Drucken zwischen etwa 1 und 100 bar. Ein Zusatz von Katalysatoren kann günstig sein, z. B. kann eine HCN-Anlagerung durch Zusatz von Palladium-bis-[2,3-O-isopropyliden-2,3-dihydroxy-1,4-bis-(diphenylphosphino)-butan] katalysiert werden.

Beispielsweise können Ester der Formel II ($R^{1'}$ und/oder $R^{2'}$ = -O-COR und/oder -COOR und/oder $Z^0$ und/oder $Z^{1'}$ = -CO-O- oder -O-CO-) durch Veresterung entsprechender Carbonsäuren z. B. der Formeln:

R-COOH,
$R^{1'}$-A-COOH,
$R^{1'}$-A-$Z^0$-$(A^{1'})_m$-COOH oder
$R^{1'}$-A-$Z^0$-$(A^{1'})_m$-$A^{2'}$-COOH

(oder ihrer reaktionsfähigen Derivate) mit Alkoholen bzw. Phenolen der Formeln

$R^{1'}$-A-$Z^0$-$(A^{1'}$-$Z^{1'})_m$-$(A^{2'})_n$-OH,
$R^{2'}$-$(A^{2'})_n$-$(Z^{1'}$-$A^{1'})_m$-$Z^0$-A-OH,
$R^{2'}$-$(A^{2'})_n$-$(Z^{1'}$-$A^{1'})_m$-OH,
$R^{2'}$-$(A^{2'})_n$-OH oder
$R^{2'}$-$(A^{2'})_{n-1}$-OH

(oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, z. B. auch gemischte Anhydride der Formeln

$R^{1'}$-A-CO-O-$COCH_3$,
$R^{1'}$-A-$Z^0$-$(A^{1'}$-$Z^{1'})_m$-CO-O-$COCH_3$ oder
$R^{1'}$-A-$Z^0$-$(A^{1'}$-$Z^{1'})_m$-$(A^{2'})_n$-CO-O-$COCH_3$,
Azide oder Ester, insbesondere Alkylester mit 1 - 4 C-Atomen in der Alkylgruppe.

Als reaktionsfähige Derivate der genannten Alkohole bzw. Phenole kommen insbesondere die entsprechenden Metallalkoholate bzw. Phenolate der Formeln:

$R^{1'}$-A-$Z^0$-$(A^{1'}$-$Z^{1'})_m$-$(A^{2'})_n$-OM,
$R^{2'}$-$(A^{2'})_n$-$(Z^{1'}$-$A^{1'})_m$-$Z^0$-A-OM,
$R^{2'}$-$(A^{2'})_n$-$(Z^{1'}$-$A^{1'})_m$-OM,
$R^{2'}$-$(A^{2'})_n$-OM oder
$R^{2'}$-$(A^{2'})_{n-1}$-OM

in Betracht, worin M ein Äquivalent eines Metalls, vorzugsweise eines Alkalimetalls wie Na oder K, bedeutet.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z. B. Pyridin, Chinolin oder Triethylamin als Lösungsmittel für die Veresterung angewandt werden. Die Veresterung kann auch in Abwesenheit eines Lösungsmittels, z. B. durch einfaches Erhitzen der Komponenten in Gegenwart von Natriumacetat, durchgeführt werden. Die Reaktionstemperatur liegt gewöhnlich zwischen -50° und +250°, vorzugsweise zwischen -20° und +80°C. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbomat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin vom Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z. B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa -25° und +20°C. Beispielsweise können Dioxanderivate bzw. Dithianderivate der Formel II (worin eine der Gruppen $A^{1'}$ und/oder $A^{2'}$ eine 1,3-Dioxan-2,5-diyl-Gruppe bzw. 1,3-Dithian-2,5-diyl-Gruppe bedeutet) zweckmäßig durch Reaktion eines entsprechenden Aldehyds, z. B. der Formeln:

$R^{1'}$-A-$Z^0$-$(A^{1'}$-$Z^{1'})_m$-$A^{2'}$-CHO,
$R^{1'}$-A-$Z^0$-$(A^{1'})_m$-CHO bzw.
O=CH-$R^{2'}$

(oder eines seiner reaktionsfähigen Derivate) mit einem entsprechenden 1,3-Diol z. B. der Formeln:

$(HOCH_2)_2CH$-$(A^{2'})_{n-2}$-$R^{2'}$,
$(HOCH_2)_2CH$-$(A^{2'})_{n-1}$-$R^{2'}$ bzw.
$R^{1'}$-A-$Z^0$-$(A^{1'}$-$Z^{1'})_m$-$(A^{2'})_{n-1}$-$CH(CH_2OH)_2$

(oder einem seiner reaktionsfähigen Derivate) bzw. einem entsprechenden 1,3-Dithiol (oder einem seiner reaktionsfähigen Derivate) hergestellt werden, vorzugsweise in Gegenwart eines inerten Lösungsmittels wie Benzol oder Toluol und/oder eines Katalysators, z. B. einer starken Säure wie Schwefelsäure, Benzol- oder p-Toluolsulfonsäure, bei Temperaturen zwischen etwa 20° und etwa 150°, vorzugsweise zwischen 80° und 120°C. Als reaktionsfähige Derivate der Ausgangsstoffe eignen sich in erster Linie Acetale z. B. der Formeln:

$R^{1'}$-A-$Z^0$-$(A^{1'}$-$Z^{1'})_m$-$A^{2'}$-$CH(OR^4)_2$,
$R^{1'}$-A-$Z^0$-$(A^{1'})_m$-$CH(OR^4)_2$,
$(R^4O)_2$-$R^{2'}$,
$R^{1'}$-A-$Z^0$-$(A^{1'}$-$Z^{1'})_m$-$A^{2'}$-$CH(CH_2O)_2CH$-$R^5$ bzw.

$R^{1'}$-A-$Z^0$-$(A^{1'})_m$-$CH(CH_2O)_2CHR^5$,
worin

$R^4$ Alkyl mit 1 - 4 C-Atomen,
zwei Reste $R^4$ zusammen auch Alkylen mit 2 oder 3 C-Atomen und
$R^5$ H, Alkyl mit 1 - 4 C-Atomen oder Phenyl bedeuten.

Die genannten Aldehyde und 1,3-Diole bzw. 1,3-Dithiole sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester und die Dithiole durch Umsetzung entsprechender Dihalogenide mit NaSH erhältlich.

Erfindungsgemäßer Ether (worin $R^{1'}$ und/oder $R^{2'}$ eine Alkylgruppe bedeuten, worin eine oder zwei $CH_2$-Gruppen durch O-Atome ersetzt sind, und/oder worin $Z^0$ und/oder $Z^{1'}$ eine $-OCH_2-$ oder eine $-CH_2O-$Gruppe sind) sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z. B. durch

Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen etwa 20° und 100°C.

Zur Herstellung erfindungsgemäßen von Verbindungen, worin $R^{1'}$ und/oder $R^{2'}$ SOR bzw. $SO_2R$ bedeuten, kann man entsprechende Verbindungen, worin $R^{1'}$ und/oder $R^{2'}$ SR bzw. SOR bedeuten, oxidieren.

Man oxidiert je nach dem gewählten Reagenz und den angewendeten Bedingungen zu den entsprechenden Sulfoxiden oder zu den entsprechenden Sulfonen, wobei man nach an sich aus der Literatur bekannten Methoden arbeitet und die Reaktionsbedingungen im einzelnen aus der Literatur leicht entnehmen kann. Will man die Sulfoxide erhalten, so oxidiert man die entsprechenden Thioether beispielsweise mit Wasserstoffperoxid, Persäuren, Cr(VI)-Verbindungen wie Chromsäure, Salpetersäure, nitrosen Gasen, $N_2O_3$, Halogenen wie Chlor, Hypochloriten, $KMnO_4$, N-Bromsuccinimid, 1-Chlorbenztriazol, Ce(IV)-Verbindungen wie $(NH_4)_2Ce(NO_3)_6$, negativ substituierten aromatischen Diazoniumsalzen wie o- oder p-Nitrophenyldiazoniumchlorid oder elektrolytisch unter verhältnismäßig milden Bedingungen und bei relativ niedrigen Temperaturen (etwa -80 bis +100°C). Will man dagegen die Sulfone erhalten, so verwendet man die gleichen Oxidationsmittel unter kräftigeren Bedingungen und/oder im Überschuß sowie in der Regel bei höheren Temperaturen. Bei diesen Umsetzungen können die üblichen inerten Lösungsmittel zugegen oder abwesend sein. Als inerte Lösungsmittel eignen sich beispielsweise Wasser, wässrige Mineralsäuren, wässrige Alkalilaugen, niedrigere Alkohole wie Methanol oder Ethanol, Ester wie Ethylacetat, Ketone wie Aceton, niedere Carbonsäuren wie Essigsäure, Nitrile wie Acetonitril, Kohlenwasserstoffe wie Benzol, chlorierte Kohlenwasserstoffe wie Chloroform oder $CCl_4$.

Ein bevorzugtes Oxidationsmittel ist 30-%-iges wässriges Wasserstoffperoxid. Dieses führt bei Anwendung der berechneten Menge in Lösungsmittel wie Essigsäure, Aceton, Ethanol oder wässriger Natronlauge bei Temperaturen zwischen -20 und 100°C zu den Sulfoxiden, im Überschuß bei höheren Temperaturen, vorzugsweise in Essigsäure oder in einem Gemisch aus Essigsäure und Acetanhydrid, zu den Sulfonen.

Ein weiteres bevorzugtes Oxidationsmittel ist 3-Chlorperbenzoesäure. Diese führt bei Anwendung der berechneten Menge in Lösungsmitteln wie Halogenkohlenwasserstoffen bei Temperaturen unterhalb von 0° in der Regel zu, den Sulfoxiden, im Überschuß bei Temperaturen zwischen 0° und Raumtemperatur zu den Sulfonen.

Eine weitere Möglichkeit zur Herstellung der Sulfoxide besteht darin, daß man die Thioether mit Chlor, z. B. in feuchtem Benzol oder in Essigsäure, behandelt. Die intermediär erhaltenen Dichlorverbindungen werden durch Hydrolyse sehr leicht in die Sulfoxide umgewandelt.

Die Thiother werden nach an sich bekannten Methoden hergestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Vorzugsweise werden die Thioether durch Behandlung entsprechender Halogenverbindungen, worin Halogen Chlor, Brom oder Jod bedeutet, mit Salzen entsprechender Mercaptane erhalten.

Diese Halogenverbindungen sind entweder bekannt, oder sie können ohne Schwierigkeiten nach an sich bekannten Methoden in Analogie zu bekannten Verbindungen hergestellt werden. So sind beispielsweise p-substituierte Halogenbenzolderivate durch Halogenierung der entsprechenden Benzolderivate zugänglich. 4-substituierte Cyclohexylhalogenide sind beispielsweise durch Reduktion der entsprechenden 4-substituierten Cyclohexanone zu den 4-substituierten Cyclohexanolen und nachfolgender Substitution durch Halogenid erhältlich.

Bei der Synthese der Halogenverbindungen können im Prinzip alle Methoden angewendet werden, die für die Verbindungen bekannt sind, die an Stelle des Halogens andere Substituenten tragen. Der Fachmann kann die erforderlichen Synthesevarianten nach Routinemethoden ableiten.

Zur Herstellung von erfindungsgemäßen Verbindungen worin A eine in 1- oder 4-Stellung durch eine Alkylgruppe mit 1 - 5 C-Atomen, worin eine oder zwei nicht benachbarte $CH_2$-Gruppen durch -S-, -SO- oder $-SO_2-$ ersetzt sind, substituierte 1,4-Cyclohexylengruppe bedeutet, können die analogen Methoden wie zur Herstellung der Verbindungen mit schwefelhaltigen Gruppen $R^1$, $R^2$ und/oder $R^3$ angewendet werden.

Zur Herstellung von erfindungsgemäßen Verbindungen die $CF_3$-Gruppen enthalten, kann man entsprechende Carbonsäuren, die ihrerseits z. B. durch Hydrolyse entsprechender Nitrile erhältlich sind, mit $SF_4$ umsetzen, zweckmäßig mit einem Überschuß von $SF_4$ unter Druck in Abwesenheit oder in Gegenwart eines inerten Lösungsmittels wie Cyclohexan oder Dichlormethan bei Temperaturen zwischen etwa 70 und 200°C. Die Reaktionszeiten bewegen sich zwischen etwa 2 Stunden und etwa 4 Tagen.

Nitrile der Formel II, worin A eine durch CN substituierte 1,4-Cyclohexylengruppe bedeutet, sind auch durch Alkylierung von Acetonitrilen der Formel II mit 1,5-Di-$X^1$-pentanderivaten der Formel III erhältlich. Die Acetonitrile sind beispielsweise aus entsprechenden Halogeniden der Formel

$E^1-CH_2X^1$

und Metallcyaniden erhältlich, die Verbindungen III durch Reduktion entsprechender Glutarsäurediester zu den entsprechenden Diolen (III, $X^1=OH$) sowie gegebenenfalls Umsetzung derselben mit anorganischen Halogeniden wie $SOCl_2$, HBr oder HJ. Das Acetonitril wird zweckmäßig zunächst mit einer starken Base wie

NaH, $NaNH_2$, Lithiumdiisopropylamid, -piperidid oder -2,5-diisopropyl-piperidid oder K-tert.Butylat in das entsprechende Carbanion übergeführt, vorzugsweise in einem inerten Lösungsmittel, beispielsweise einem Kohlenwasserstoff wie Toluol, einem Ether wie THF oder Dioxan, einem Amid wie DMF, einem Sulfoxid wie Dimethylsulfoxid oder einem Gemisch derartiger Lösungsmittel. Nach Zugabe von III (worin $X^1$ von OH verschieden ist) hält man zweckmäßig 0,5 bis 16 Stunden bei Temperaturen zwischen 0° und 150°C. Eine Umsetzung von II mit III ($X^1$ = OH) gelingt dagegen zweckmäßig in Gegenwart von Azodicarbonsäureestern/Triphenylphosphin in THF bei Temperaturen zwischen etwa -30° und +30°C.

In ganz analoger Weise sind Nitrile der Formel II, worin A eine in 1- oder 4-Stellung durch CN substituierte 1,4-Cyclohexylengruppe bedeutet, die zusätzlich durch 1 - 2 F-Atome und/oder CN-Gruppen substituiert sein kann, erhältlich, indem man ein Nitril der Formel IV mit einem Halogenid der Formel V umsetzt. Die Nitrile der Formel IV sind beispielsweise aus entsprechenden Amiden der Formel

$Q^1$-$A^{3'}CONH_2$

durch Dehydratisierung erhältlich, die Halogenide der Formel V aus entsprechenden Alkoholen der Formel

$Q^2$-OH.

Eine Base der Formel II kann mit einer Säure in das zugehörige Säureadditionssalz übergeführt werden. Für diese Umsetzung können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -di-sulfonsäuren, Laurylschwefelsäure.

Umgekehrt ist es möglich, aus einem Säureadditionssalz einer Verbindung der Formel II die Base der Formel II durch Behandeln mit einer Base freizusetzen, z. B. mit einer starken anorganischen Base wie KOH oder NaOH.

Bevorzugte Zwischenprodukte zur Herstellung der erfindungsgemäßen Verbindungen der Formel II sind die Cyclohexanone der Formeln (A) und (B),

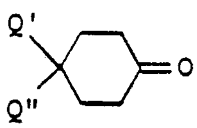

(A)

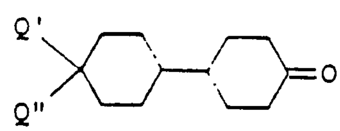

(B)

worin

Q' eine Alkylgruppe mit 3 - 15 C-Atomen, worin auch eine oder zwei nicht benachbarte $CH_2$-Gruppen durch eine Gruppierung aus der Gruppe -O-, -CO-, -S-, -SO- und -$SO_2$- ersetzt sein können,

Q'' COOR°, OR°, SR°, SOR°, $SO_2R°$, R°, $CF_3$, $CCl_3$, $CHF_2$, CN, CHO, F, Cl oder Br, und

R° eine Alkylgruppe mit 1 - 3 C-Atomen

bedeutet, sowie deren reaktionsfähige Derivate.

Besonders bevorzugte Zwischenprodukte sind diejenigen Verbindungen der Formeln (A) und (B), worin

Q' eine geradkettige Alkylgruppe mit 4 - 9 C-Atomen und

Q'' -CN, -CHO, -$CF_3$, -$CH_3$, $OCH_3$-, -$SCH_3$, -$SOCH_3$, -$SO_2CH_3$, -$COOCH_3$ oder F, insbesondere -CN, -$CH_3$ oder F,

bedeutet.

Die Verbindungen der Formeln (A) und (B) werden nach an sich bekannten Methoden hergestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Verbindungen der Formeln (A) und (B) lassen sich beispielsweise durch Reduktion der Carbonylgruppe und anschließende Veresterung in Esterderivate der Formel II überführen. Durch Veretherung der aus (A) und (B) durch Reduktion erhaltenen Alkohole erhältlich sind Etherderivate der Formel II.

Eine Vielzahl von Verbindungen der Formel II sind durch Grignard-Reaktion mit den Cyclohexanonen der Formeln (A) und (B) und nachfolgender Reduktion erhältlich. Wittig-Reaktion mit 2-substituierten 1-Bromethanderivaten/Triphenylphosphin und nachfolgende Hydrierung liefert eine Vielzahl von Verbindungen der Formel II mit $-CH_2CH_2-$Gruppen zwischen zwei Ringstrukturen.

Die erfindungsgemäßen flüssigkristallinen Phasen bestehen aus 2 bis 15, vorzugsweise 3 bis 12 Komponenten, darunter mindestens einer erfindungsgemäßen Verbindung. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Biscyclohexylbiphenyle, Phenyl- oder Cyclohexylpyrimidine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, gegebenenfalls halogenierten Stilbene, Benzylphenylether, Tolane und substituierten Zimtsäuren.

Weiterhin bevorzugt sind erfindungsgemäße Phasen, die mindestens eine Verbindung der Formel II enthalten.

Die wichtigsten als Bestandteile derartiger flüssigkristalliner Phasen in Frage kommenden Verbindungen lassen sich durch die Formel IX charakterisieren,

$$R^6\text{-}L\text{-}G\text{-}E\text{-}R^7 \qquad IX$$

worin

L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | -CH=CH- | -N(O)=N- |
| | -CH=CY- | -CH=N(O)- |
| | -C≡C- | $-CH_2-CH_2-$ |
| | -CO-O- | $-CH_2-O-$ |
| | -CO-S- | $-CH_2-S-$ |
| | -CH=N- | -COO-Phe-COO- |

oder eine C-C-Einfachbindung,

Y Halogen, vorzugsweise Chlor, oder -CN, und

$R^6$ und $R^7$ Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br

bedeuten.

Bei den meisten dieser Verbindungen sind $R^6$ und $R^7$ voneinander verschieden, wobei einer dieser Reste meist eine Alkyl- oder Alkoxygruppe ist. Aber auch andere Varianten der vorgesehenem Substituenten sind gebräuchlich. Viele solcher Substanzen oder auch Gemische davon sind im Handel erhältlich. Alle diese Substanzen sind nach literaturbekannten Methoden erhältlich.

Die erfindungsgemäßen Phasen enthalten etwa 0,1 bis 99, vorzugsweise 10 bis 95 %, einer oder mehrerer erfindungsgemäßen Verbindungen. Weiterhin bevorzugt sind erfindungsgemäße flüssigkristalline Fhasen, enthaltend 0,1 - 40, vorzugsweise 0,5 - 30 % einer oder mehrerer erfindungsgemäße Verbindungen.

Die Herstellung der erfindungsgemäßen Phasen erfolgt in an sich üblicher Weise. In der Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammoniumtetraphenylboranat oder Komplexsalze von Kronenethern (vgl. z. B. I. Haller et al., Mol. Cryst. Liq. Cryst., Band 24, (1973) Seiten 249 - 258) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z. B. in den DE-OS-2 209 127, 2 240 864, 2 321 632, 2 338 281, 2 450 088, 2 637 430, 2 853 728 und 2 902 177 beschrieben.

Die folgenden Beispiele sollen die Erfindung erläutern, ohne sie zu begrenzen.

F. = Schmelzpunkt,

K. = Klärpunkt.

Vor- und nachstehend bedeuten Prozentangaben Gewichtsprozent;

alle Temperaturen sind in Grad Celsius angegeben.

"Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

**Beispiel 1**

Zu 10,1 g (0,1 mol) Diisopropylamin in 70 ml THF werden unter Feuchtigkeitsausschluß und Stickstoffatmosphäre bei -10° nacheinander 62,5 ml (0,1 mol) einer 1,6 m Lösung von n-Butyllithium in Hexan und 33,1 g (0,1 mol) 4-(4-Cyanocyclohexyl)-4'-pentylbiphenyl (erhältlich durch Grignard-Reaktion von 4-Brom-4'-pentyl-biphenyl mit 4-(4,5-Dihydro-4,4-dimethyl-2-oxazolyl)-cyclohexanon (J. Org. Chem. 39 (18), (1974) 2737-93), Abspaltung von Wasser und gleichzeitiger Entfernung der Oxazolylschutzgruppe durch Kochen mit ethanolischer $H_2SO_4$, Hydrierung der Doppelbindung und anschließender Überführung des Esters in das Nitril) in 40 ml THF getropft. Dann wird das Reaktionsgemisch 20 Minuten gerührt. Anschließend gibt man ebenfalls bei -10° 13,5 g (0,11 mol) 1-Brompropan zu und rührt dann nochmals 20 Minuten bei Raumtemperatur. Nach üblicher Aufarbeitung erhält man 4-(4-Cyano-4-propylcyclohexyl)-4'-pentylbiphenyl.

Analog werden hergestellt:

4-(4-Cyano-4-propylcyclohexyl)-4'-ethylbiphenyl
4-(4-Cyano-4-propylcyclohexyl)-4'-propylbiphenyl
4-(4-Cyano-4-propylcyclohexyl)-4'-butylbiphenyl
4-(4-Cyano-4-propylcyclohexyl)-4'-heptylbiphenyl

4-(4-Cyano-4-butylcyclohexyl)-4'-ethylbiphenyl
4-(4-Cyano-4-butylcyclohexyl)-4'-propylbiphenyl
4-(4-Cyano-4-butylcyclohexyl)-4'-butylbiphenyl
4-(4-Cyano-4-butylcyclohexyl)-4'-pentylbiphenyl
4-(4-Cyano-4-butylcyclohexyl)-4'-heptylbiphenyl

4-(4-Cyano-4-pentylcyclohexyl)-4'-ethylbiphenmyl
4-(4-Cyano-4-pentylcyclohexyl)-4'-propylbiphenyl
4-(4-Cyano-4-pentylcyclohexyl)-4'-butylbiphenyl
4-(4-Cyano-4-pentylcyclohexyl)-4'-pentylbiphenyl
4-(4-Cyano-4-pentylcyclohexyl)-4'-heptylbiphenyl

4-(4-Cyano-4-heptylcyclohexyl)-4'-ethylbiphenyl
4-(4-Cyano-4-heptylcyclohexyl)-4'-propylbiphenyl
4-(4-Cyano-4-heptylcyclohexyl)-4'-butylbiphenyl
4-(4-Cyano-4-heptylcyclohexyl)-4'-pentylbiphenyl
4-(4-Cyano-4-heptylcyclohexyl)-4'-heptylbiphenyl

4-(4-Cyano-4-nonylcyclohexyl)-4'-ethylbiphenyl
4-(4-Cyano-4-nonylcyclohexyl)-4'-propylbiphenyl
4-(4-Cyano-4-nonylcyclohexyl)-4'-butylbiphenyl
4-(4-Cyano-4-nonylcyclohexyl)-4'-pentylbiphenyl
4-(4-Cyano-4-nonylcyclohexyl)-4'-heptylbiphenyl

**Beipiel 2**

Aus 4-(4-Cyanocyclohexyl)-4'-(4-pentylcyclohexyl)-biphenyl (erhältlich aus 4-Brom-4'-(4-pentylcylohexyl)-biphenyl analog Beispiel 1) und 1-Brompropan erhält man analog zu Beispiel 1 4-(4-Cyano-4-propylcyclohexyl)-4'-(4-pentylcyclohexyl)-biphenyl.

Analog werden hergestellt:

4-(4-Cyano-4-propylcyclohexyl)-4'-(4-ethylcyclohexyl)-biphenyl
4-(4-Cyano-4-propylcyclohexyl)-4'-(4-propylcyclohexyl)-biphenyl
4-(4-Cyano-4-propylcyclohexyl)-4'-(4-butylcyclohexyl)-biphenyl
4-(4-Cyano-4-propylcyclohexyl)-4'-(4-heptylcyclohexyl)-biphenyl

4-(4-Cyano-4-butylcyclohexyl)-4'-(4-ethylcyclohexyl)-biphenyl
4-(4-Cyano-4-butylcyclohexyl)-4'-(4-propylcyclohexyl)-biphenyl
4-(4-Cyano-4-butylcyclohexyl)-4'-(4-butylcyclohexyl)-biphenyl
4-(4-Cyano-4-butylcyclohexyl)-4'-(4-pentylcyclohexyl)-biphenyl
4-(4-Cyano-4-butylcyclohexyl)-4'-(4-heptylcyclohexyl)-biphenyl

4-(4-Cyano-4-pentylcyclohexyl)-4'-(4-ethylcyclohexyl)-biphenyl
4-(4-Cyano-4-pentylcyclohexyl)-4'-(4-propylcyclohexyl)-biphenyl
4-(4-Cyano-4-pentylcyclohexyl)-4'-(4-butylcyclohexyl)-biphenyl

4-(4-Cyano-4-pentylcyclohexyl)-4'-(4-pentylcyclohexyl)-biphenyl
4-(4-Cyano-4-pentylcyclohexyl)-4'-(4-heptylcyclohexyl)-biphenyl

4-(4-Cyano-4-heptylcyclohexyl)-4'-(4-ethylcyclohexyl)-biphenyl
4-(4-Cyano-4-heptylcyclohexyl)-4'-(4-propylcyclohexyl)-biphenyl
4-(4-Cyano-4-heptylcyclohexyl)-4'-(4-butylcyclohexyl)-biphenyl
4-(4-Cyano-4-heptylcyclohexyl)-4'-(4-pentylcyclohexyl)-biphenyl
4-(4-Cyano-4-heptylcyclohexyl)-4'-(4-heptylcyclohexyl)-biphenyl

**Beipiel 3**

Zu einer Lösung von 0,05 mol 4-(4-Cyano-4-propyl-cyclohexyl)-4'-(4-pentylcyclohexyl)-biphenyl (Beispiel 2) in 100 ml Hexan gibt man bei 0° unter Stickstoffatmosphäre und unter Ausschluß von Feuchtigkeit 55 ml einer 1 m Lösung von Diisobutylaluminiumhydrid (DIBAH) in n-Hexan. Man rührt eine Stunde bei Raumtemperatur und gibt dann 5 ml Methanol vorsichtig zu. Das Reaktionsgemisch wird in 200 ml eiskalte 10-%-ige $H_2SO_4$ gegossen und 30 Minuten gerührt. Dann wird die organische Phase abgetrennt und die wäßrige Phase noch zweimal mit Hexan extrahiert. Die vereinigten org. Phasen werden mit $NaHCO_3$-Lösung neutralisiert und dann getrocknet. Nach chromatographischer Reinigung erhält man 4-(4-Formyl-4-propylcyclohexyl)-4'-(4-pentylcyclohexyl)-biphenyl.

Analog werden hergestellt:

4-(4-Formyl-4-propylcyclohexyl)-4'-(4-ethylcyclohexyl)-biphenyl.
4-(4-Formyl-4-propylcyclohexyl)-4'-(4-propylcyclohexyl)-biphenyl.
4-(4-Formyl-4-propylcyclohexyl)-4'-(4-butylcyclohexyl)-biphenyl.
4-(4-Formyl-4-propylcyclohexyl)-4'-(4-heptylcyclohexyl)-biphenyl.

4-(4-Formyl-4-butylcyclohexyl)-4'-(4-ethylcyclohexyl)-biphenyl.
4-(4-Formyl-4-butylcyclohexyl)-4'-(4-propylcyclohexyl)-biphenyl.
4-(4-Formyl-4-butylcyclohexyl)-4'-(4-butylcyclohexyl)-biphenyl.
4-(4-Formyl-4-butylcyclohexyl)-4'-(4-pentylcyclohexyl)-biphenyl.
4-(4-Formyl-4-butylcyclohexyl)-4'-(4-heptylcyclohexyl)-biphenyl.

4-(4-Formyl-4-pentylcyclohexyl)-4'-(4-ethylcyclohexyl)-biphenyl.
4-(4-Formyl-4-pentylcyclohexyl)-4'-(4-propylcyclohexyl)-biphenyl.
4-(4-Formyl-4-pentylcyclohexyl)-4'-(4-butylcyclohexyl)-biphenyl.
4-(4-Formyl-4-pentylcyclohexyl)-4'-(4-pentylcyclohexyl)-biphenyl.
4-(4-Formyl-4-pentylcyclohexyl)-4'-(4-heptylcyclohexyl)-biphenyl.

4-(4-Formyl-4-heptylcyclohexyl)-4'-(4-ethylcyclohexyl)-biphenyl.
4-(4-Formyl-4-heptylcyclohexyl)-4'-(4-propylcyclohexyl)-biphenyl.
4-(4-Formyl-4-heptylcyclohexyl)-4'-(4-butylcyclohexyl)-biphenyl.
4-(4-Formyl-4-heptylcyclohexyl)-4'-(4-pentylcyclohexyl)-biphenyl.
4-(4-Formyl-4-heptylcyclohexyl)-4'-(4-heptylcyclohexyl)-biphenyl.

**Beispiel 4**

Eine Mischung aus 0,01 mol 4-(4-Formyl-4-propylcyclohexyl)-4'-(4-pentylcyclohexyl)-biphenyl (Beispiel 3), 40 ml Diethylenglykol, 15 ml 90-%-iges Hydrazinhydrat und 5,5 g Kaliumhydroxid wird erhitzt und daraus Hydrazin und Wasser abdestilliert bis die Temperatur auf etwa 230° gestiegen ist. Dann wird 12 Stunden gekocht und anschließend zu der kalten Reaktionsmischung 200 ml Wasser gegeben. Nach üblicher Aufarbeitung erhält man 4-(4-Methyl-4-propylcyclohexyl)-4'-(4-pentylcyclohexyl)-biphenyl.

Analog werden hergestellt:

4-(4-Methyl-4-propylcyclohexyl)-4'-(4-ethylcyclohexyl)-biphenyl
4-(4-Methyl-4-propylcyclohexyl)-4'-(4-propylcyclohexyl)-biphenyl
4-(4-Methyl-4-propylcyciohexyl)-4'-(4-butylcyclohexyl)-biphenyl
4-(4-Methyl-4-propylcyclohexyl)-4'-(4-heptylcyclohexyl)-biphenyl

4-(4-Methyl-4-butylcyclohexyl)-4'-(4-ethylcyclohexyl)-biphenyl
4-(4-Methyl-4-butylcyclohexyl)-4'-(4-propylcyclohexyl)-biphenyl

4-(4-Methyl-4-butylcyclohexyl)-4'-(4-butylcyclohexyl)-biphenyl
4-(4-Methyl-4-butylcyclohexyl)-4'-(4-pentylcyclohexyl)-biphenyl
4-(4-Methyl-4-butylcyclohexyl)-4'-(4-heptylcyclohexyl)-biphenyl

4-(4-Methyl-4-pentylcyclohexyl)-4'-(4-ethylcyclohexyl)-biphenyl
4-(4-Methyl-4-pentylcyclohexyl)-4'-(4-propylcyclohexyl)-biphenyl
4-(4-Methyl-4-pentylcyclohexyl)-4'-(4-butylcyclohexyl)-biphenyl
4-(4-Methyl-4-pentylcyclohexyl)-4'-(4-pentylcyclohexyl)-biphenyl
4-(4-Methyl-4-pentylcyclohexyl)-4'-(4-heptylcyclohexyl)-biphenyl

4-(4-Methyl-4-heptylcyclohexyl)-4'-(4-ethylcyclohexyl)-biphenyl
4-(4-Methyl-4-heptylcyclohexyl)-4'-(4-propylcyclohexyl)-biphenyl
4-(4-Methyl-4-heptylcyclohexyl)-4'-(4-butylcyclohexyl)-biphenyl
4-(4-Methyl-4-heptylcyclohexyl)-4'-(4-pentylcyclohexyl)-biphenyl
4-(4-Methyl-4-heptylcyclohexyl)-4'-(4-heptylcyclohexyl)-biphenyl

**Beipiel 5**

Zu 10,1 g (0,1 mol) Diisopropylamin in 100 ml THF werden unter Feuchtigkeitsausschuß und Stickstoffatmosphäre bei -10° nacheinander 62,5 ml (0,1 mol) einer 1,6 m Lösung von n-Butyllithium in Hexan und 36,5 g (0,1 mol) 1-(p-(4-Cyanocyclohexyl)-phenyl)-2-(trans-4-pentylcyclohexyl)-ethan [erhältlich durch Friedel-Crafts-Acylierung von Benzol mit trans-4-Pentylcyclohexylacetylchlorid, Reduktion der Carbonylgruppe nach Wolff-Kishner, Bromierung des Aromaten in p-Stellung und anschließende Grignard-Reaktion des so erhaltenen 1-(p-Bromphenyl)-2-(trans-4-pentylcyclohexyl)-ethans mit 4-(4,5-Dihydro-4,4-dimethyl-2-oxazoyl)-cyclohexanon, Abspaltung von Wasser unter gleichzeitiger Entfernung der Oxazolylschutzgruppe durch Kochen mit ethanolischer $H_2SO_4$, Hydrierung der Doppelbindung und Überführung des Ethylesters in das Nitril] in 50 ml THF getropft. Das Reaktionsgemisch wird 20 Minuten gerührt. Anschließend gibt man dazu bei -10° 16,6 g (0,11 mol) Brompentan und rührt dann nochmals 20 Minuten bei Raumtemperatur. Nach üblicher Aufarbeitung erhält man 1-[p-(4-Cyano-4-pentylcyclohexyl)-phenyl]-2-(trans-4-pentylcyclohexyl)-ethan.

Analog werden hergestellt:

1-[p-(4-Cyano-4-pentylcyclohexyl)-phenyl]-2-(trans-4-ethylcyclohexyl)-ethan.
1-[p-(4-Cyano-4-pentylcyclohexyl)-phenyl]-2-(trans-4-propylcyclohexyl)-ethan.
1-[p-(4-Cyano-4-pentylcyclohexyl)-phenyl]-2-(trans-4-butylcyclohexyl)-ethan.
1-[p-(4-Cyano-4-pentylcyclohexyl)-phenyl]-2-(trans-4-heptylcyclohexyl)-ethan.

1-[p-(4-Cyano-4-heptylcyclohexyl)-phenyl]-2-(trans-4-ethylcyclohexyl)-ethan.
1-[p-(4-Cyano-4-heptylcyclohexyl)-phenyl]-2-(trans-4-propylcyclohexyl)-ethan.
1-[p-(4-Cyano-4-heptylcyclohexyl)-phenyl]-2-(trans-4-butylcyclohexyl)-ethan.
1-[p-(4-Cyano-4-heptylcyclohexyl)-phenyl]-2-(trans-4-pentylcyclohexyl)-ethan.
1-[p-(4-Cyano-4-heptylcyclohexyl)-phenyl]-2-(trans-4-heptylcyclohexyl)-ethan.

1-[p-(4-Cyano-4-butylcyclohexyl)-phenyl]-2-(trans-4-ethylcyclohexyl)-ethan.
1-[p-(4-Cyano-4-butylcyclohexyl)-phenyl]-2-(trans-4-propylcyclohexyl)-ethan.
1-[p-(4-Cyano-4-butylcyclohexyl)-phenyl]-2-(trans-4-butylcyclohexyl)-ethan.
1-[p-(4-Cyano-4-butylcyclohexyl)-phenyl]-2-(trans-4-pentylcyclohexyl)-ethan.
1-[p-(4-Cyano-4-butylcyclohexyl)-phenyl]-2-(trans-4-heptylcyclohexyl)-ethan.

1-[p-(4-Cyano-4-propylcyclohexyl)-phenyl]-2-(trans-4-ethylcyclohexyl)-ethan.
1-[p-(4-Cyano-4-propylcyclohexyl)-phenyl]-2-(trans-4-propylcyclohexyl)-ethan.
1-[p-(4-Cyano-4-propylcyclohexyl)-phenyl]-2-(trans-4-butylcyclohexyl)-ethan.
1-[p-(4-Cyano-4-propylcyclohexyl)-phenyl]-2-(trans-4-pentylcyclohexyl)-ethan.
1-[p-(4-Cyano-4-propylcyclohexyl)-phenyl]-2-(trans-4-heptylcyclohexyl)-ethan.

1-[p-(4-Cyano-4-ethylcyclohexyl)-phenyl]-2-(trans-4-ethylcyclohexyl)-ethan.
1-[p-(4-Cyano-4-ethylcyclohexyl)-phenyl)-2-(trans-4-propylcyclohexyl)-ethan.
1-[p-(4-Cyano-4-ethylcyclohexyl)-phenyl]-2-(trans-4-butylcyclohexyl)-ethan.
1-[p-(4-Cyano-4-ethylcyclohexyl)-phenyl]-2-(trans-4-pentylcyclohexyl)-ethan.
1-[p-(4-Cyano-4-ethylcyclohexyl)-phenyl]-2-(trans-4-heptylcyclohexyl)-ethan.

**Beipiel 6**

Aus 1-[p-(4-Cyano-4-pentylcyclohexyl)-phenyl]-2-(trans-4-pentylcyclohexyl)-ethan (Beipiel 5) erhält man analog zu Beispiel 3 1-[p-(4-Formyl-4-pentylcyclohexyl)-phenyl]-2-(trans-4-pentylcyclohexyl)-ethan.
Analog werden hergestellt:

1-[p-(4-Formyl-4-pentylcyclohexyl)-phenyl]-2-(trans-4-ethylcyclohexyl)-ethan
1-[p-(4-Formyl-4-pentylcyclohexyl)-phenyl]-2-(trans-4-propylcyclohexyl)-ethan
1-[p-(4-Formyl-4-pentylcyclohexyl)-phenyl]-2-(trans-4-butylcyclohexyl)-ethan
1-[p-(4-Formyl-4-pentylcyclohexyl)-phenyl]-2-(trans-4-heptylcyclohexyl)-ethan

1-[p-(4-Formyl-4-heptylcyclohexyl)-phenyl]-2-(trans-4-ethylcyclohexyl)-ethan
1-[p-(4-Formyl-4-heptylcyclohexyl)-phenyl]-2-(trans-4-propylcyclohexyl)-ethan
1-[p-(4-Formyl-4-heptylcyclohexyl)-phenyl]-2-(trans-4-butylcyclohexyl)-ethan
1-[p-(4-Formyl-4-heptylcyclohexyl)-phenyl]-2-(trans-4-pentylcyclohexyl)-ethan
1-[p-(4-Formyl-4-heptylcyclohexyl)-phenyl]-2-(trans-4-heptylcyclohexyl)-ethan

1-[p-(4-Formyl-4-butylcyclohexyl)-phenyl]-2-(trans-4-ethylcyclohexyl)-ethan
1-[p-(4-Formyl-4-butylcyclohexyl)-phenyl]-2-(trans-4-propylcyclohexyl)-ethan
1-[p-(4-Formyl-4-butylcyclohexyl)-phenyl]-2-(trans-4-butylcyclohexyl)-ethan
1-[p-(4-Formyl-4-butylcyclohexyl)-phenyl]-2-(trans-4-pentylcyclohexyl)-ethan
1-[p-(4-Formyl-4-butylcyclohexyl)-phenyl]-2-(trans-4-heptylcyclohexyl)-ethan

1-[p-(4-Formyl-4-propylcyclohexyl)-phenyl]-2-(trans-4-ethylcyclohexyl)-ethan
1-[p-(4-Formyl-4-propylcyclohexyl)-phenyl]-2-(trans-4-propylcyclohexyl)-ethan
1-[p-(4-Formyl-4-propylcyclohexyl)-phenyl]-2-(trans-4-butylcyclohexyl)-ethan
1-[p-(4-Formyl-4-propylcyclohexyl)-phenyl]-2-(trans-4-pentylcyclohexyl)-ethan
1-[p-(4-Formyl-4-propylcyclohexyl)-phenyl]-2-(trans-4-heptylcyclohexyl)-ethan

**Beispiel 7**

Eine Mischung aus 4,4 g (0,01 mol) 1-[p-(4-Formyl-4-pentylcyclohexyl)-phenyl]-2-(trans-4-pentylcyclohexyl)-ethan (Beispiel 6), 40 ml Diethylenglykol, 15 ml 90 % Hydrazin und 5,5 g Kaliumhydroxid wird erhitzt und daraus Hydrazin und Wasser abdestilliert bis die Temperatur auf etwa 230° gestiegen ist. Dann wird 12 Stunden gekocht und anschließend zu der kalten Reaktionsmischung 200 ml Wasser gegeben. Nach üblicher Aufarbeitung erhält man 1-[p-(4-Methyl-4-pentylcyclohexyl)-phenyl]-2-(trans-4-pentylcyclohexyl)-ethan.
Analog werden hergestellt:

1-[p-(4-Methyl-4-pentylcyclohexyl)-phenyl]-2-(trans-4-ethylcyclohexyl)-ethan
1-[p-(4-Methyl-4-pentylcyclohexyl)-phenyl]-2-(trans-4-propylcyclohexyl)-ethan
1-[p-(4-Methyl-4-pentylcyclohexyl)-phenyl]-2-(trans-4-butylcyclohexyl)-ethan
1-[p-(4-Methyl-4-pentylcyclohexyl)-phenyl]-2-(trans-4-heptylcyclohexyl)-ethan

1-[p-(4-Methyl-4-heptylcyclohexyl)-phenyl]-2-(trans-4-ethylcyclohexyl)-ethan
1-[p-(4-Methyl-4-heptylcyclohexyl)-phenyl]-2-(trans-4-propylcyclohexyl)-ethan
1-[p-(4-Methyl-4-heptylcyclohexyl)-phenyl]-2-(trans-4-butylcyclohexyl)-ethan
1-[p-(4-Methyl-4-heptylcyclohexyl)-phenyl]-2-(trans-4-pentylcyclohexyl)-ethan
1-[p-(4-Methyl-4-heptylcyclohexyl)-phenyl]-2-(trans-4-heptylcyclohexyl)-ethan

1-[p-(4-Methyl-4-butylcyclohexyl)-phenyl]-2-(trans-4-ethylcyclohexyl)-ethan
1-[p-(4-Methyl-4-butylcyclohexyl)-phenyl]-2-(trans-4-propylcyclohexyl)-ethan
1-[p-(4-Methyl-4-butylcyclohexyl)-phenyl]-2-(trans-4-butylcyclohexyl)-ethan
1-[p-(4-Methyl-4-butylcyclohexyl)-phenyl]-2-(trans-4-pentylcyclohexyl)-ethan
1-[p-(4-Methyl-4-butylcyclohexyl)-phenyl]-2-(trans-4-heptylcyclohexyl)-ethan

1-[p-(4-Methyl-4-propylcyclohexyl)-phenyl]-2-(trans-4-ethylcyclohexyl)-ethan
1-[p-(4-Methyl-4-propylcyclohexyl)-phenyl)-2-(trans-4-propylcyclohexyl)-ethan
1-[p-(4-Methyl-4-propylcyclohexyl)-phenyl]-2-(trans-4-butylcyclohexyl)-ethan
1-[p-(4-Methyl-4-propylcyclohexyl)-phenyl]-2-(trans-4-pentylcyclohexyl)-ethan
1-[p-(4-Methyl-4-propylcyclohexyl)-phenyl]-2-(trans-4-heptylcyclohexyl)-ethan

1-[p-(4-Methyl-4-ethylcyclohexyl)-phenyl]-2-(trans-4-ethylcyclohexyl)-ethan
1-[p-(4-Methyl-4-ethylcyclohexyl)-phenyl]-2-(trans-4-propylcyclohexyl)-ethan

1-[p-(4-Methyl-4-ethylcyclohexyl)-phenyll-2-(trans-4-butylcyclohexyl)-ethan
1-[p-(4-Methyl-4-ethylcyclohexyl)-phenyl]-2-(trans-4-pentylcyclohexyl)-ethan
1-[p-(4-Methyl-4-ethylcyclohexyl)-phenyl]-2-(trans-4-heptylcyclohexyl)-ethan

**Beispiel 8**

Zu 19,8 g (0,1 mol) trans-4-Pentylcyclohexancarbonsäure in 75 ml $CH_2Cl_2$ werden unter Rühren 1 g 4-Dimethyl-aminopyridin und 30,3 g (0,1 mol) 4-Cyano-4-heptyl-4'-hydroxybicyclohexan [erhältlich aus 4-(1,4-Dioxaspiro[4,5]dec-8-yl)-cyclohexanon durch Einführung der Nitrilgruppe mit Tosylmethylisocyanid (TOSMIC), Alkylierung mit LDA (Diisopropylamin/Butyllithium) und Heptylbromid, Abspaltung der Keto-Schutzgruppe, Reduktion des Ketons mit $NaBH_4$ zum Alkohol und Isomerentrennung] gegeben.

Unter Temperaturkontrolle tropft man dazu bei 0° eine Lösung von 27,7 g (0,105 mol) Dicyclohexylcarbodiimid in 50 ml Methylenchlorid und rührt dann noch eine Stunde bei Raumtemperatur. Der ausgefallene Harnstoff wird abgetrennt und die Methylenchloridphase mit verdünnter HCl und gesättigter $NaHCO_3$-Lösung gewaschen und getrocknet. Anschließend wird das Produkt durch Filtration über eine kurze Kieselgelsäule und durch Kristallisation gereinigt. Man erhält 4-Cyano-4-heptyl-4'-(trans-4-pentylcyclohexylcarbonyloxy)-bicyclohexan.

Analog werden hergestellt:

4-Cyano-4-heptyl-4'-(trans-4-ethylcyclohexylcarbonyloxy)-bicyclohexan
4-Cyano-4-heptyl-4'-(trans-4-propylcyclohexylcarbonyloxy)-bicyclohexan
4-Cyano-4-heptyl-4'-(trans-4-butylcyclohexylcarbonyloxy)-bicyclohexan
4-Cyano-4-heptyl-4'-(trans-4-heptylcyclohexylcarbonyloxy)-bicyclohexan

4-Cyano-4-pentyl-4'-(trans-4-ethylcyclohexylcarbonyloxy)-bicyclohexan
4-Cyano-4-pentyl-4'-(trans-4-propylcyclohexylcarbonyloxy)-bicyclohexan
4-Cyano-4-pentyl-4'-(trans-4-butylcyclohexylcarbonyloxy)-bicyclohexan
4-Cyano-4-pentyl-4'-(trans-4-pentylcyclohexylcarbonyloxy)-bicyclohexan
4-Cyano-4-pentyl-4'-(trans-4-heptylcyclohexylcarbonyloxy)-bicyclohexan
4-Cyano-4-propyl-4'-(trans-4-ethylcyclohexylcarbonyloxy)-bicyclohexan
4-Cyano-4-propyl-4'-(trans-4-propylcyclohexylcarbonyloxy)-bicyclohexan
4-Cyano-4-propyl-4'-(trans-4-butylcyclohexylcarbonyloxy)-bicyclohexan
4-Cyano-4-propyl-4'-(trans-4-pentylcyclohexylcarbonyloxy)-bicyclohexan
4-Cyano-4-propyl-4'-(trans-4-heptylcyclohexylcarbonyloxy)-bicyclohexan

4-Cyano-4-ethyl-4'-(trans-4-ethylcyclohexylcarbonyloxy)-bicyclohexan
4-Cyano-4-ethyl-4'-(trans-4-propylcyclohexylcarbonyloxy)-bicyclohexan
4-Cyano-4-ethyl-4'-(trans-4-butylcyclohexylcarbonyloxy)-bicyclohexan
4-Cyano-4-ethyl-4'-(trans-4-pentylcyclohexylcarbonyloxy)-bicyclohexan
4-Cyano-4-ethyl-4'-(trans-4-heptylcyclohexylcarbonyloxy)-bicyclohexan

**Beispiel 9**

Ein Isomerengemisch aus 1-Hydroxy-1-[4'-(trans-4-pentylcyclohexyl)-biphenyl-4-yl]-4-n-propylcyclohexan [erhältlich durch Umsetzung von 4-n-Propylcyclohexanon mit 4'-(trans-4-pentylcyclohexyl)-biphenyl-4-yl-magnesiumbromid und nachfolgenden Hydrolyse] wird chromatographisch in die beiden Stereoisomeren aufgetrennt.

4,5 g eines dieser Alkohole werden in 150 ml Methylenchlorid gelöst und innerhalb 20 min bei -60° zu einer Lösung von 1,7 ml Diethylaminoschwefeltrifluorid (DAST) und 50 ml Methylenchlorid getropft. Nach 5 Stunden wird das Gemisch auf Raumtemperatur erwarmen gelassen und 8 Stunden gerührt. Nach üblicher Aufarbeitung und Umkristallisation aus Ethylacetat erhält man 2,2 g r-1-Fluor-1-[4'-(trans-4-pentylcyclohexyl)-biphenyl-4-yl]-trans-4-propylcyclohexan, F. 267°.

In analoger Weise erhält man aus 2,0 g des anderen Alkohols 0,3 g r-1-Fluor-1-[4'-(trans-4-pentylcyclohexyl)-biphenyl-4-yl]-cis-4-propylcyclohexan, F. 176°, K. 309°.

Analog werden aus den entsprechenden Alkoholen hergestellt (jeweils beide Stereoisomere):

1-Fluor-1-ethyl-4-[4'-(trans-4-pentylcyclohexyl)-biphenyl-4-yl]-cyclohexan
1-Fluor-1-propyl-4-[4'-(trans-4-pentylcyclohexyl)-biphenyl-4-yl]-cyclohexan
1-Fluor-1-butyl-4-[4'-(trans-4-pentylcyclohexyl)-biphenyl-4-yl]-cyclohexan
1-Fluor-1-pentyl-4-[4'-(trans-4-pentylcyclohexyl)-biphenyl-4-yl]-cyclohexan
1-Fluor-1-heptyl-4-[4'-(trans-4-pentylcyclohexyl)-biphenyl-4-yl]-cyclohexan

1-Fluor-1-[4'-trans-4-pentylcyclohexyl)-biphenyl-4-yl]-4-ethylcyclohexan
1-Fluor-1-[4'-trans-4-pentylcyclohexyl)-biphenyl-4-yl]-4-propylcyclohexan
1-Fluor-1-[4'-trans-4-pentylcyclohexyl)-biphenyl-4-yl]-4-butylcyclohexan
1-Fluor-1-[4'-trans-4-pentylcyclchexyl)-biphenyl-4-yl]-4-pentylcyclohexan
1-Fluor-1-[4'-trans-4-pentylcyclohexyl)-biphenyl-4-yl]-4-heptylcyclohexan

**Beispiel 10**

Eine Lösung von 26,3 g 4'-Pentyl-biphenylyl-4-acetonitril [F. 80°, K. -10°; erhältlich aus 4-Acetyl-4'-pentyl-biphenyl über 4'-Pentyl-biphenylyl-4-essigsäure (F. 160°) und das entsprechende Amid (F. 185°)] in 150 ml Dimethylsulfoxid wird unter Rühren mit 6 g NaH (50-%-ig in Paraffin), dann mit einer Lösung von 1-Brom-3-(2-bromethyl)-n-hexan in 50 ml Dioxan versetzt; dabei hält man die Temperatur durch Kühlen unter 35°. Man rührt noch 2 Std., versetzt mit Isopropanol und arbeitet wie üblich auf. Man erhält r-1-Cyan-1-(4'-pentyl-4-biphenylyl)-cis-4-propylcyclohexan.

Analog erhält man aus den entsprechenden Acetonitrilen und den entsprechenden Dibromiden:

r-1-Cyan-1-(4'-pentyl-4-biphenylyl)-cis-4-ethylcyclohexan
r-1-Cyan-1-(4'-pentyl-4-biphenylyl)-cis-4-butylcyclohexan
r-1-Cyan-1-(4'-pentyl-4-biphenylyl)-cis-4-pentylcyclohexan
r-1-Cyan-1-(4'-pentyl-4-biphenylyl)-cis-4-heptylcyclohexan

r-1-Cyan-1-(4'-propyl-4-biphenylyl)-cis-4-ethylcyclohexan
r-1-Cyan-1-(4'-propyl-4-biphenylyl)-cis-4-propylcyclohexan
r-1-Cyan-1-(4'-propyl-4-biphenylyl)-cis-4-butylcyclohexan
r-1-Cyan-1-(4'-propyl-4-biphenylyl)-cis-4-pentylcyclohexan
r-1-Cyan-1-(4'-propyl-4-biphenylyl)-cis-4-heptylcyclohexan

r-1-Cyan-1-(4'-butyl-4-biphenylyl)-cis-4-ethylcyclohexan
r-1-Cyan-1-(4'-butyl-4-biphenylyl)-cis-4-propylcyclohexan
r-1-Cyan-1-(4'-butyl-4-biphenylyl)-cis-4-butylcyclohexan
r-1-Cyan-1-(4'-butyl-4-biphenylyl)-cis-4-pentylcyclohexan
r-1-Cyan-1-(4'-butyl-4-biphenylyl)-cis-4-heptylcyclohexan

r-1-Cyan-1-(4'-heptyl-4-biphenylyl)-cis-4-ethylcyclohexan
r-1-Cyan-1-(4'-heptyl-4-biphenylyl)-cis-4-propylcyclohexan
r-1-Cyan-1-(4'-heptyl-4-biphenylyl)-cis-4-butylcyclohexan
r-1-Cyan-1-(4'-heptyl-4-biphenylyl)-cis-4-pentylcyclohexan
r-1-Cyan-1-(4'-heptyl-4-biphenylyl)-cis-4-heptylcyclohexan.

**Beispiel 11**

In eine Lösung von 61 g p-(4-Methyl-4-n-pentyl-cyclohexyl)-benzoylchlorid [erhältlich durch Umsetzung des aus 2-Methylheptanal und Pyrrolidin erhältlichen Enamins mit Methylvinylketon nach G. Otani und S. Yamada, Chem. Pharm. Bull. 21 (10), (1973) 2112-2118, Hydrierung des erhaltenen 4-Methyl-4-pentyl-2-cyclohexenon zum 4-Methyl-4-pentyl-cyclohexanon (ein Zwischenprodukt der Formel (A)), Umsetzung mit Phenylmagnesiumbromid, Hydrolyse und Hydrierung zum 4-Methyl-4-n-pentylcyclohexyl-benzol, Umsetzung mit Acetylchlorid in Gegenwart von Aluminiumchlorid, nachfolgenden Haloformabbau des erhaltenen Acetophenons zur entsprechenden Benzoesäure und Überführung in das Säurenchlorid] in 40 ml Toluol tropft man eine Lösung von 2,7 g 4-Cyan-3-fluorphenol und 5 ml Pyridin in 30 ml Toluol und kocht 2 Stunden. Nach üblicher Aufarbeitung erhält man p-(4-Methyl-4-n-pentylcyclohexyl)-benzoesäure-(4-cyan-3-fluorphenylester).

Analog werden hergestellt:

p-(4-Methyl-4-ethylcyclohexyl)-benzoesäure-(4-cyan-3-fluorphenylester)
p-(4-Methyl-4-propylcyclohexyl)-benzoesäure-(4-cyan-3-fluorphenylester)
p-(4-Methyl-4-butylcyclohexyl)-benzoesäure-(4-cyan-3-fluorphenylester)
p-(4-Methyl-4-hexylcyclohexyl)-benzoesäure-(4-cyan-3-fluorphenylester)
p-(4-Methyl-4-heptylcyclohexyl)-benzoesäure-(4-cyan-3-fluorphenylester)

Die folgenden Beispiele betreffen erfindungsgemäße flüssigkristalline Phasen.

**Beispiel A**

Man stellt eine flüssigkristalline Phase her aus:
15 % 2-p-Cyanphenyl-5-propyl-1,3-dioxan,
23 % 2-p-Cyanphenyl-5-butyl-1,3-dioxan,
17 % 2-p-Cyanphenyl-5-pentyl-1,3-dioxan,
7 % 4-(4-Cyan-4-propylcyclohexyl)-4'-pentylbiphenyl,
12 % 4-Cyan-4-heptyl-4'-(trans-4-pentyl-cyclohexyl-carbonyloxy)-bicyclohexan,
11 % 4-Propyl-4'-heptyl-bicyclohexyl-4'-carbonsäure-methylester,
6 % p-trans-4-Ethylcyclohexyl-benzoesäure-(p-cyan-phenylester),
7 % p-trans-4-Pentylcyclohexyl-benzoesäure-(p-cyan-phenylester) und
2 % p-(p-Propylbenzoyloxy)-benzoesäure-(p-cyan-phenylester).

**Beispiel B**

Man stellt eine flüssigkristalline Phase her aus:
9 % trans-4-Propylcyclohexancarbonsäure-(trans-4-propyl-cyclohexylester),
7 % trans-4-Pentylcyclohexancarbonsäure-(trans-4-propyl-cyclohexylester),
17 % 2-p-Cyanphenyl-5-propyl-1,3-dioxan,
25 % 2-p-Cyanphenyl-5-butyl-1,3-dioxan,
18 % 2-p-Cyanphenyl-5-pentyl-1,3-dioxan,
7 % 1-Fluor-1-[4'-(trans-4-pentylcyclohexyl)-biphenyl-4-yl]-4-propylcyclohexan,
3 % 1-Fluor-1-[4'-(trans-4-propylcyclohexyl)-biphenyl-4-yl]-4-propylcyclohexan,
5 % 4-(trans-4-Pentylcyclohexyl)-4'-(trans-4-propylcyclohexyl)-biphenyl und
9 % p-Methoxybenzoesäure-(p-pentylphenylester).

**Patentansprüche**

1. Cyclohexanderivate der Formel II

$$R^{1'}\text{-}A\text{-}Z^0\text{-}(A^{1'}\text{-}Z^{1'})_m\text{-}(A^{2'})_n\text{-}R^{2'} \qquad II$$

worin

| | |
|---|---|
| $R^{1'}$ und $R^{2'}$ | jeweils H, eine Alkylgruppe mit 1 - 10 C-Atomen worin auch eine oder zwei nicht benachbarte $CH_2$-Gruppen durch O-Atome oder -CH=CH- ersetzt sein können, F, Cl, Br, CN, -COOR, -O-COR, SOR, $SO_2R$ oder $-C\equiv C-R^{3'}$, |
| $A^{1'}$ und $A^{2'}$ | jeweils unsubstituierte oder durch ein oder zwei F- und/oder Cl-Atome und/oder $CH_3$-Gruppen und/oder CN-Gruppen substituierte 1,4-Phenylen-, 1,4-Cyclohexylen-, 1,3-Dioxan-2,5-diyl-, 1,3-Dithian-2,5-diyl-, Piperidin-1,4-diyl-, 1,4-Bicyclo(2,2,2)octylen-, Pyridazin-3,6-diyl- oder Pyrimidin-2,5-diylgruppen oder A, |
| A | eine in 1- und/oder 4-Stellung durch unsubstituiertes oder substituiertes Alkyl oder fluoriertes Alkyl mit jeweils 1 - 5 C-Atomen, worin auch eine oder zwei nicht benachbarte $CH_2$-Gruppen durch eine Gruppierung aus der Gruppe -O-, -CO-, -O-CO-, -CO-O-, $-C\equiv C-$, -CH=CH-, -S-, -SO- und $-SO_2-$ ersetzt sein können, F, Cl, Br, CN und/oder -CHO substituierte 1,4-Cyclohexylen-Gruppe, die 1 oder 2 weitere Substituenten tragen kann, |
| $Z^0$ und $Z^{1'}$ | jeweils -CO-O-, -O-CO-, $-CH_2CH_2-$, $-OCH_2-$, $-CH_2O-$ oder eine Einfachbindung, |
| $R^{3'}$ | H, CN oder eine Alkylgruppe mit 1 - 7 C-Atomen, |
| R | eine Alkylgruppe mit 1 - 10 C-Atomen, und |
| m und n | jeweils unabhängig voneinander 1 oder 2 |

bedeuten, und
$(m + n) \geqq 3$, oder falls $Z^0$ eine Einfachbindung bedeutet und $Z^{1'}$ keine Einfachbindung bedeutet, auch $\geqq 2$ ist, wobei für
m = 2 und/oder n = 2 die Gruppen $A^{1'}$ und/oder $A^{2'}$ und/oder $Z^{1'}$ gleich oder voneinander verschieden sein können,
sowie Cyclohexanderivate der Formeln

| | |
|---|---|
| Alkyl-Phe-Phe-A-Alkyl | VIII 32 |
| Alkoxy-Phe-Phe-A-Alkyl | VIII 33 |
| Alkyl-Phe-Phe-CO-O-A-Alkyl | VIII 53 |

Alkoxy-Phe-Phe-CO-O-A-Alkyl VIII 54
Alkyl-Phe-Phe-O-CO-A-Alkyl VIII 55
Alkoxy-Phe-Phe-O-CO-A-Alkyl VIII 56

worin -A

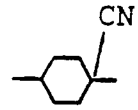

und Phe jeweils unsubstituiertes oder durch ein oder zwei F- und/oder Cl-Atome und/oder $CH_3$-Gruppen und/oder CN-Gruppen substituiertes 1,4-Phenylen bedeutet
und Alkyl und Alkoxy 1 bis 10 C-Atome haben.

2. Verfahren zur Herstellung von Cyclohexanderivaten der Formel II, sowie Cyclohexanderivaten der Formeln VIII 32, VIII 33, VIII 53, VIII 54, VIII 55 und VIII 56, dadurch gekennzeichnet, daß man eine Verbindung, die sonst der Formel II entspricht, aber an Stelle von H-Atomen eine oder mehrere reduzierbare Gruppen und/oder C-C-Bindungen enthält, mit einem reduzierenden Mittel behandelt,

oder daß man an eine Verbindung, die sonst der Formel II entspricht, aber an Stelle des Restes A eine 1-Cyclohexen-1,4-diylgruppe enthält, die 1 oder 2 weitere F-, Cl- oder Br-Atome und/oder CN-Gruppen tragen kann, eine Verbindung der Formel HX (worin X F, Cl, Br oder CN bedeutet) anlagert,

oder daß man zur Herstellung von Estern der Formel II (worin $R^{1'}$ und/oder $R^{2'}$ -OCOR und/oder -COOR bedeuten und/oder worin $Z^0$ und/oder $Z^{1'}$ -CO-O- oder -O-CO- bedeuten), VIII 53, VIII 54, VIII 55 oder VIII 56 eine entsprechende Carbonsäure oder eines ihrer reaktionsfähigen Derivate mit einem entsprechenden Alkohol oder einem seiner reaktionsfähigen Derivate umsetzt,

oder daß man zur Herstellung von Dioxanderivaten bzw. Dithianderivaten der Formel II (worin $A^{1'}$ und/oder $A^{2'}$ 1,3-Dioxan-2,5-diyl bzw. 1,3-Dithian-2,5-diyl bedeuten) einen entsprechenden Aldehyd oder eines seiner reaktionsfähigen Derivate mit einem entsprechenden Diol bzw. Dithiol umsetzt,

oder daß man zur Herstellung von Nitrilen der Formel II (worin $R^{1'}$ und/oder $R^{2'}$ CN bedeuten und/oder worin A und/oder $A^{1'}$ und/oder $A^{2'}$ durch mindestens eine CN-Gruppe substituiert sind) ein entsprechendes Carbonsäureamid dehydratisiert oder ein entsprechendes Carbonsäurehalogenid mit Sulfamid umsetzt,

oder daß man zur Herstellung von Nitrilen der Formel II (worin A eine in 1- oder 4-Stellung durch CN substituierte 1,4-Cyclohexylengruppe bedeutet) ein Acetonitril der Formel III

$E^{1'}$-$CH_2CN$ III

worin
$E^1$ a) $R^{1'}$- oder
b) $R^{2'}$-$(A^{2'})_n$-$(Z^{1'}$-$A^{1'})_m$-$Z^0$-
bedeutet und
$R^{1'}$, $R^{2'}$, $A^{1'}$, $A^{2'}$, $Z^{1'}$, $Z^0$, m und n die angegebenen Bedeutungen haben,
mit einer Verbindung der Formel IV

$(X^1$-$CH_2$-$CH_2)_2CH$-$E^2$ IV

worin
$X^1$ Cl, Br, J, OH oder eine reaktionsfähig veresterte OH-Gruppe bedeutet,
$E^2$ a) $R^{2'}$-$(A^{2'})_n$-$(Z^{1'}$-$A^{1'})_m$-$Z^0$- oder
b) $R^{1'}$-
bedeutet und
$R^{1'}$, $R^{2'}$, $A^{1'}$, $A^{2'}$, $Z^{1'}$, $Z^0$, m und n die angegebenen Bedeutungen haben,
umsetzt,

oder daß man zur Herstellung von Nitrilen der Formel II (worin A eine in 1- oder 4-Stellung durch CN substituierte 1,4-Cyclohexylengruppe bedeutet, die zusätzlich durch ein oder zwei F-Atome und/oder CN-Gruppen substituiert Sein kann; worin ferner bei (a) $Z^0$ eine Einfachbindung bedeutet) ein Nitril der Formel V

$Q^1$-$Q^3$-CN V

worin
$Q^1$ a) $R^{1'}$- oder
b) $R^{2'}$-$(A^{2'})_n$-$(Z^{1'}$-$A^{1'})_m$-$Z^0$- und
$Q^3$ eine unsubstituierte oder eine ein- oder zweifach durch F und/oder CN substituierte 1,4-Cyclohexylengruppe
bedeuten und
$R^{1'}$, $R^{2'}$, $A^{1'}$, $A^{2'}$, $Z^{1'}$, $Z^0$, m und n die angegebenen Bedeutungen haben,
mit einer Verbindung der Formel VI

$Q^2$-$X^1$ VI

worin

$Q^2$ a) $R^{2'}$-$(A^{2'})_n$-$(Z^{1'}$-$A^{1'})_m$- oder

b) $R^{1'}$-

bedeuten und

$X^{1'}$, $R^{1'}$, $R^{2'}$, $A^{1'}$, $A^{2'}$, $Z^{1'}$, m und n die angegebenen Bedeutungen haben,

umsetzt,

oder daß man zur Herstellung von Ethern der Formel II (worin $R^{1'}$ und/oder $R^{2'}$ eine Alkylgruppe bedeuten, worin eine oder zwei $CH_2$-Gruppen durch O-Atome ersetzt sind und/oder $Z^0$ und/oder $Z^{1'}$ eine $-OCH_2-$ oder $-CH_2O-$Gruppe sind) eine entsprechende Hydroxyverbindung verethert,

oder daß man zur Herstellung von Verbindungen der Formel II, worin $R^{1'}$ und/oder $R^{2'}$ SOR bzw $SO_2R$ bedeuten, eine entsprechende Verbindung worin $R^1$ und/oder $R^2$ SR bzw. SOR bedeuten, oxidiert,

oder daß man zur Herstellung von Verbindungen der Formel II, die $CF_3$-Gruppen enthalten, eine entsprechende Carbonsaure mit $SF_4$ umsetzt,

und/oder daß man gegegenenfalls eine Chlor- oder Bromverbindung der Formel II (worin $R^{1'}$ und/oder $R^{2'}$ Cl oder Br bedeuten und/oder worin A durch mindestens ein Chlor- oder Bromatom substituiert ist und/oder $A^{1'}$ und/oder $A^{2'}$ durch mindestens ein Chloratom substituiert sind) mit einem Cyanid umsetzt,

und/oder daß man gegebenenfalls eine Base der Formel II durch Behandeln mit einer Säure in eines ihrer Säureadditionssalze umwandelt,

oder daß man gegebenenfalls eine Verbindung der Formel II aus einem ihrer Säureadditionssalze durch Behandeln mit einer Base freisetzt,

oder daß man zur Herstellung von Nitrilen der Formeln VIII 32 oder VIII 33 ein entsprechendes 4-substituiertes Cyclohexancarbonitril mit einer entsprechenden Halogenverbindung, Hydroxyverbindung oder einem ihrer reaktionsfähigen Derivate umsetzt.

3. Verwendung der Verbindungen der Formel II sowie VIII 32, VIII 33, VIII 53, VIII 54, VIII 55 und VIII 56 nach Anspruch 1 als Komponenten flüssigkristalliner Phasen.

4. Flüssigkristalline Phasen mit mindestens zwei flüssigkristallinen Komponenten, dadurch gekennzeichnet, daß mindestens eine Komponente eine Verbindung der Formel II, VIII 32, VIII 33, VIII 53, VIII 54, VIII 55 oder VIII 56 nach Anspruch 1 ist.

5. Flüssigkristall-Anzeigeelement, dadurch gekennzeichnet, daß es eine flüssigkristalline Phase nach Anspruch 4 enthält.

6. Elektrooptisches Anzeigeelement nach Anspruch 5, dadurch gekennzeichnet, daß es als Dielektrikum eine flüssigkristalline Phase nach Anspruch 4 enthält.

**Claims**

1. Cyclohexane derivatives of the formula II

$$R^1\text{-}A\text{-}Z^0\text{-}(A^{1'}\text{-}Z^{1'})_m\text{-}(A^{2'})_n\text{-}R^{2'} \qquad \text{II}$$

wherein

$R^{1'}$ and $R^{2'}$ are each H, an alkyl group with 1 - 10 C atoms, it also being possible for one or two non-adjacent $CH_2$ groups to be replaced by O atoms or by $-CH=CH-$, F, Cl, Br, CN, -COOR, -O-COR, SOR, $SO_2R$ or $-C\equiv C-R^{3'}$,

$A^{1'}$ and $A^{2'}$ are each 1,4-phenylene, 1,4-cyclohexylene, 1,3-dioxane-2,5-diyl, 1,3-dithiane-2,5-diyl, piperidine-1,4-diyl, 1,4-bicyclo(2,2,2)-octylene, pyridazine-3,6-diyl or pyrimidine-2,5-diyl groups which are unsubstituted or substituted by one or two F and/or Cl atoms and/or $CH_3$ groups and/or CN groups, or A,

A is a 1,4-cyclohexylene group which is substituted in the 1- and/or 4-position by unsubstituted or substituted alkyl or fluorinated alkyl with in each case 1 - 5 C atoms, it also being possible for one or two non-adjacent $CH_2$ groups to be replaced by a grouping from the group comprising -O-, -CO-, -O-CO-, -CO-O-, $-C\equiv C-$, -S-, -SO- and $-SO_2-$, or by F, Cl, Br, CN and/or -CHO, and which can carry 1 or 2 further substituents,

$Z^0$ and $Z^{1'}$ are each -CO-O-, -O-CO-, $-CH_2CH_2-$, $-OCH_2-$, $-CH_2O-$ or a single bond,

$R^{3'}$ is H, CN or an alkyl group with 1 - 7 C atoms,

R is an alkyl group with 1 - 10 C atoms and

m and n are each independently of one another 1 or 2,

and

$(m + n) \geq 3$ or $\geq 2$, if $Z^0$ is a single bond and $Z^{1'}$ is not a single bond, wherein, for m = 2 and/or n = 2, the groups $A^{1'}$ and/or $A^{2'}$ and/or $Z^{1'}$ can be identical or different, as well as cyclohexane derivatives of the formulae

Alkyl-Phe-Phe-A-Alkyl VIII 32

Alkoxy-Phe-Phe-A-Alkyl VIII 33
Alkyl-Phe-Phe-CO-O-A-Alkyl VIII 53
Alkoxy-Phe-Phe-CO-O-A-Alkyl VIII 54
Alkyl-Phe-Phe-O-CO-A-Alkyl VIII 55
Alkoxy-Phe-Phe-O-CO-A-Alkyl VIII 56

wherein

-A- is 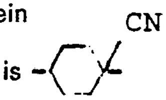

and Phe is in each case 1,4-phenylene which is unsubstituted or substituted by one or two F and/or Cl atoms and/or $CH_3$ groups and/or CN groups,

and Alkyl and Alkoxy each have 1 to 10 C atoms.

2. Process for the preparation of cyclohexane derivatives of the formula II as well as cyclohexane derivatives of formulae VIII 32, VIII 33, VIII 53, VIII 54, VIII 55 and VIII 56, characterized in that a compound which otherwise corresponds to the formula II but contains one or more reducible groups and/or C-C bonds instead of H atoms is treated with a reducing agent,

or in that a compound of the formula HX (wherein X is F, Cl, Br or CN) is added onto a compound which otherwise corresponds to the formula II but, instead of the radical A, contains a 1-cyclohexene-1,4-diyl group which can carry 1 or 2 further F, Cl or Br atoms and/or CN groups,

or in that, to prepare esters of the formula II (wherein $R^{1'}$ and/or $R^{2'}$ are -OCOR and/or -COOR and/or wherein $Z^0$ and/or $Z^{1'}$ are -CO-O- or -O-CO-), VIII 53, VIII 54, VIII 55 or VIII 56 a corresponding carboxylic acid or one of its reactive derivatives is reacted with a corresponding alcohol or one of its reactive derivatives,

or in that, to prepare dioxane derivatives or dithiane derivatives of the formula II (wherein $A^{1'}$ and/or $A^{2'}$ are 1,3-dioxane-2,5-diyl or 1,3-dithiane-2,5-diyl), a corresponding aldehyde or one of its reactive derivate is reacted with a corresponding diol or dithiol,

or in that, to prepare nitriles of the formula II (wherein $R^{1'}$ and/or $R^{2'}$ are CN and/or wherein A and/or $A^{1'}$ and/or $A^{2'}$ are substituted by at least one CN group), a corresponding carboxylic acid amide is dehydrated or a corresponding carboxylic acid halide is reacted with sulfamide,

or in that, to prepare nitriles of the formula II (wherein A is a 1,4-cyclohexylene group which is substituted in the 1- or 4-position by CN), an acetonitrile of the formula III

$E^1$-$CH_2CN$ III

wherein

$E^1$ is a) $R^{1'}$- or

b) $R^{2'}$-$(A^{2'})_n$-$(Z^{1'}$-$A^{1'})_m$-$Z^0$-

and

$R^{1'}$, $R^{2'}$, $A^{1'}$, $A^{2'}$, $Z^{1'}$, $Z^0$, m and n have the meanings given,

is reacted with a compound of the formula IV

$(X^1$-$CH_2$-$CH_2)_2CH$-$E^2$ IV

wherein

$X^1$ is Cl, Br, I, OH or a reactive esterified OH group,

$E^2$ is a) $R^{2'}$-$(A^{2'})_n$-$(Z^{1'}$-$A^{1'})_m$-$Z^0$- or

b) $R^1$-

and

$R^{1'}$, $R^{2'}$, $A^{1'}$, $A^{2'}$, $Z^{1'}$, $Z^0$, m and n have the meanings given,

or in that, to prepare nitriles of the formula II (wherein A is a 1,4-cyclohexylene group which is substituted in the 1- or 4-position by CN and which can additionally be substituted by one or two F atoms and/or CN groups; and wherein, furthermore, in (a) $Z^0$ is a single bond), a nitrile of the formula V

$Q^1$-$Q^3$-CN V

wherein

$Q^1$ is (a) $R^{1'}$- or

(b) $R^{2'}$-$(A^{2'})_n$-$(Z^{1'}$-$A^{1'})_m$-$Z^0$- and

$Q^3$ is a 1,4-cyclohexylene group which is unsubstituted or mono- or di-substituted by F and/or CN,

and $R^{1'}$, $R^{2'}$, $A^{1'}$, $A^{2'}$, $Z^{1'}$, $Z^0$, m and n have the meanings given,

is reacted with a compound of the formula VI

$Q^2$-$X^1$ VI

wherein

$Q^2$ is a) $R^{2'}$-$(A^{2'})_n$-$(Z^{1'}$-$A^{1'})_m$- or
b) $R^{1'}$-

and $X^{1'}$, $R^{1'}$, $R^{2'}$, $A^{1'}$, $A^{2'}$, $Z^{1'}$, m and n have the meanings given,

or in that to prepare ethers of the formula II (wherein $R^{1'}$ and/or $R^{2'}$ are an alkyl group, in which one or two $CH_2$ groups are replaced by O atoms, and/or $Z^0$ and/or $Z^{1'}$ are an $-OCH_2-$ or $-CH_2O-$ group), a corresponding hydroxy compound is etherified,

or in that, to prepare compounds of the formula II wherein $R^{1'}$ and/or $R^{2'}$ is SOR or $SO_2R$, a corresponding compound wherein $R^1$ and/or $R^2$ are SR or SOR is oxidized,

or in that, to prepare compounds of the formula II which contain $CF_3$ groups, a corresponding carboxylic acid is reacted with $SF_4$,

and/or in that, if appropriate, a chlorine or bromine compound of the formula II (wherein $R^{1'}$ and/or $R^{2'}$ are Cl or Br and/or wherein A is substituted by at least one chlorine or bromine atom and/or $A^{1'}$ and/or $A^{2'}$ are substituted by at least one chlorine atom) is reacted with a cyanide,

and/or in that, if appropriate, a base of the formula II is converted into one of its acid addition salts by treatment with an acid,

or in that, if appropriate, a compound of the formula II is liberated from one of its acid addition salts by treatment with a base, or in that, to prepare compounds of formulae VIII 32 or VIII 33, a corresponding 4-substituted cyclohexane carbonitrile is reacted with a corresponding halogen compound, hydroxy compound or one of their reactive derivatives.

3. Use of the compounds of the formula II as well as formulae VIII 32, VIII 33, VIII 53, VIII 54, VII 55 and VIII 56 according to Claim 1 as components of liquid crystal phases.

4. Liquid crystal phases with at least two liquid crystal components, characterized in that at least one component is a compound of the formula II or of the formulae VIII 32, VIII 33, VIII 53, VIII 54, VIII 55 and VIII 56 according to Claim 1.

5. Liquid crystal display element, characterized in that it contains a liquid crystal phase according to Claim 4.

6. Electrooptical display element according to Claim 5, characterized in that it contains a liquid crystal phase according to Claim 4 as the dielectric.

## Revendications

1. Dérivés du cyclohexane répondant à la formule II

$$R^{1'}\text{-}A\text{-}Z^0\text{-}(A^{1'}\text{-}Z^{1'})_m\text{-}(A^{2'})_n\text{-}R^{2'} \qquad II$$

dans laquelle

- $R^{1'}$ et $R^{2'}$ représentent chacun H, un groupe alkyle en C 1 - C 10 dans lequel un ou deux groupes $CH_2$ non voisins peuvent être remplacés par des atomes d'oxygène ou des groupes -CH=CH-, F, Cl, Br, CN, -COOR, -O-COR, SOR, $SO_2R$ ou $-C\equiv C-R^{3'}$,
- $A^{1'}$ et $A^{2'}$ représentent chacun un groupe 1,4-phenylène, 1,4-cyclohexylène, 1,3-dioxanne-2,5-diyle, 1,3-dithianne-2,5-diyle, pipéridine-1,4-diyle, 1,4-bicyclo(2,2,2)octylène, pyridazine-3,6-diyle ou pyrimidine-2,5-diyle non substitué ou substitué par 1 ou 2 atomes de F et/ou de Cl et/ou groupes $CH_3$ et/ou groupes CN, ou A,
- A représente un groupe 1,4-cyclohexylène substitué en position 1 et/ou 4 par des groupes alkyle non substitués ou substitués ou des groupes alkyle fluorés contenant chacun 1 à 5 atomes de carbone et dans lesquels un ou deux groupes $CH_2$ non voisins peuvent être remplacés par un groupement du type -O-, -CO-, -O-CO-, -CO-O-, $-C\equiv C-$, -CH=CH-, -S-, -SO- et $-SO_2-$, par F, Cl, Br, CN et/ou -CHO et qui peut porter un ou deux autres substituants,
- $Z^0$ et $Z^{1'}$ représentent chacun -CO-O-, -O-CO-, $-CH_2CH_2-$, $-OCH_2-$, $-CH_2O-$ ou une liaison simple,
- $R^{3'}$ représente H, CN ou un groupe alkyle en C 1 - C 7,
- R représente un groupe alkyle en C 1 - C 10, et
- m et n sont chacun, indépendamment l'un de l'autre, égaux à 1 ou 2, avec $(m + n) \geqq 3$ ou bien, lorsque $Z^0$ représente une liaison simple et $Z^{1'}$ a une signification autre qu'une liaison simple, $(m + n) \geqq 2$, étant spécifié que lorsque m = 2 et/ou n = 2, les groupes $A^{1'}$ et/ou $A^{2'}$ et/ou $Z^{1'}$ peuvent être identiques ou différents,

ainsi que les dérivés du cyclohexane de formules

| | |
|---|---|
| Alkyl-Phe-Phe-A-Alkyl | VIII 32 |
| Alcoxy-Phe-Phe-A-Alkyl | VIII 33 |
| Alkyl-Phe -Phe-CO-O-A-Alkyl | VIII 53 |
| Alcoxy-Phe-Phe-CO-O-A-Alkyl | VIII 54 |
| Alkyl-Phe-Phe-O-CO-A-Alkyl | VIII 55 |
| Alcoxy-Phe-Phe-O-CO-A-Alkyl | VIII 56 |

dans lesquelles

-A- représente

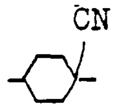

et Phe représente chacun un groupe 1,4-phénylène non substitué ou substitué par 1 ou 2 atomes de F et/ou de Cl et/ou groupes $CH_3$ et/ou groupes CN

et "Alkyl" et "Alcoxy" représentent respectivement des groupes alkyle et alcoxy en C 1 - C 10.

2. Procédé de préparation des dérivés du cyclohexane de formule II ainsi que des dérivés du cyclohexane de formules VIII 32, VIII 33, VIII 53, VIII 54, VIII 55 et VIII 56, caractérisé en ce que l'on traite par un agent reducteur un composé qui répondrait par ailleurs à la formule II mais qui contient un ou plusieurs groupes réductibles et/ou une ou plusieurs liaisons C-C réductibles à la place d'atomes d'hydrogène,

ou bien en ce que, partant d'un composé qui répondrait par ailleurs à la formule II mais qui contient à la place du groupe A un groupe 1-cyclohexène-1,4-diyle pouvant porter un ou deux autres atomes de F, de Cl ou de Br et/ou groupes CN, on fixe sur ce composé, par addition, un composé de formule HX (dans laquelle X représente F, Cl, Br ou CN),

ou bien en ce que, pour préparer les esters de formule II (dans laquelle $R^{1'}$ et/ou $R^{2'}$ représentent -OCOR et/ou -COOR et/ou dans laquelle $Z^0$ et/ou $Z^{1'}$ représentent -CO-O- ou -O-CO-), VIII 53, VIII 54, VIII 55 et VIII 56, on fait réagir un acide carboxylique correspondant ou l'un de ses derivés réactifs avec un alcool correspondant, ou l'un de ses dérivés réactifs,

ou bien en ce que, pour préparer les dérivés du dioxanne et du dithianne de formule II (dans laquelle $A^{1'}$ et/ou $A^{2'}$ représentent respectivement un groupe 1,3-dioxanne-2,5-diyle ou 1,3-dithianne-2,5-diyle), on fait réagir un aldéhyde correspondant ou l'un de ses dérivés réactifs avec respectivement un diol ou un dithiol correspondant,

ou bien en ce que, pour preparer les nitriles de formule II (dans laquelle $R^{1'}$ et/ou $R^{2'}$ représentent et/ou dans laquelle A et/ou $A^{1'}$ et/ou $A^{2'}$ sont substitués par au moins un groupe CN), on déshydrate un carboxamide correspondant ou bien on fait réagir un halogénure d'acide carboxylique correspondant avec le sulfonamide,

ou bien en ce que, pour préparer les nitriles de formule II (dans laquelle A représente un groupe 1,4-cyclohexylène substitué par CN en position 1 ou 4), on fait réagir un acétonitrile de formule III

$E^1$-$CH_2CN$ III

dans laquelle
$E^1$ représente
a) $R^{1'}$- ou
b) $R^{2'}$-$(A^{2'})_n$-$(Z^{1'}$-$A^{1'})_m$-$Z^0$
et $R^{1'}$, $R^{2'}$, $A^{1'}$, $A^{2'}$, $Z^{1'}$, $Z^0$, m et n ont les significations indiquées ci-dessus,
avec un composé de formule IV

$(X^1$-$CH_2$-$CH_2)_2CH$-$E^2$ IV

dans laquelle
$X^1$ représente Cl, Br, I, OH ou un groupe OH estérifié et réactif,
$E^2$ représente
a) $R^{2'}$-$(A^{2'})_n$-$(Z^{1'}$-$A^{1'})_m$-$Z^0$- ou
b) $R^{1'}$
et $R^{1'}$, $R^{2'}$, $A^{1'}$, $A^{2'}$, $Z^{1'}$, $Z^0$, m et n ont les significations indiquées ci-dessus,

ou bien en ce que, pour préparer les nitriles de formule II (dans laquelle A représente un groupe 1,4-cyclohexylène substitué par CN en position 1 ou 4 et qui peut en outre être substitué par un ou deux atomes de F et/ou groupes CN; dans laquelle en outre, dans le cas (a) $Z^0$ représente une liaison simple), on fait réagir un nitrile de formule V

$Q^1$-$Q^3$-CN V

dans laquelle
$Q^1$ représente
(a) $R^{1'}$- ou
(b) $R^{2'}$-$(A^{2'})_n$-$(Z^{1'}$-$A^{1'})_m$-$Z^0$- et
$Q^3$ représente un groupe 1,4-cyclohexylène non substitué ou mono- ou di-substitué par F et/ou CN, et
$R^{1'}$, $R^{2'}$, $A^{1'}$, $A^{2'}$, $Z^{1'}$, $Z^0$, m et n ont les significations indiquées ci-dessus,
avec un composé de formule VI

$Q^2$-$X^1$ VI

dans laquelle

$Q^2$ représente

a) $R^{2'}$-$(A^{2'})_n$-$(Z^{1'}$-$A^{1'})_m$- ou

b) $R^{1'}$

$X^{1'}$, $R^{1'}$, $R^{2'}$, $A^{1'}$, $A^{2'}$, $Z^{1'}$, m et n ont les significations indiquées ci-dessus,

ou bien en ce que, pour préparer les éthers de formule II (dans laquelle $R^{1'}$ et/ou $R^{2'}$ représentent un groupe alkyle dans lequel 1 ou 2 groupes $CH_2$ sont remplacés par des atomes d'oxygène et/ou $Z^0$ et/ou $Z^{1'}$ représentent un groupe $-OCH_2-$ ou $-CH_2O-$), on éthérifie un composé hydroxylé correspondant,

ou bien en ce que, pour préparer les composés de formule II dans laquelle $R^{1'}$ et/ou $R^{2'}$ représentent respectivement SOR ou $SO_2R$, on oxyde un composé correspondant dans lequel $R^1$ et/ou $R^2$ représentent respectivement SR ou SOR,

ou bien en ce que, pour préparer les composés de formule II contenant des groupes $CF_3$, on fait réagir un acide carboxylique correspondant avec $SF_4$,

et/ou en ce que l'on fait réagir le cas échéant un composé chloré ou bromé de formule II (dans laquelle $R^{1'}$ et/ou $R^{2'}$ représentent Cl ou Br et/ou dans laquelle A est substitué par au moins un atome de chlore ou de brome et/ou $A^{1'}$ et/ou $A^{2'}$ sont substitués par au moins un atome de chlore) avec un cyanure,

et/ou en ce que, le cas échéant, on convertit une base de formule II par traitement à l'aide d'un acide en l'un de ses sels formés par addition avec des acides,

ou bien en ce que, le cas échéant, on libère un composé de formule II à partir de l'un de ses sels formés par addition avec des acides en traitant par une base,

ou bien en ce que, pour préparer les nitriles de formules VIII 32 et VIII 33, on fait réagir un cyclohéxanecarbonitrile correspondant substitué en position 4 avec un composé halogéné correspondant, un composé hydroxylé correspondant ou l'un de ses dérivés réactifs.

3. Utilisation des composés de formule II et également VIII 32, VIII 33, VIII 53, VIII 54, VIII 55 et VIII 56 selon la revendication 1 en tant que composants de phases à cristaux liquides.

4. Phases à cristaux liquides contenant au moins deux composants à cristaux liquides, caractérisées en ce que l'un au moins des composants est un composé de formule II, VIII 32, VIII 33, VIII 53, VIII 54, VIII 55 ou VIII 56 selon la revendication 1.

5. Elément d'affichage à cristaux liquides caractérisé en ce qu'il contient une phase à cristaux liquides selon la revendication 4.

6. Elément d'affichage électro-optique selon la revendication 5, caractérisé en ce qu'il contient en tant que diélectrique une phase à cristaux liquides selon la revendication 4.